(19) Europäisches Patentamt
European Patent Office
Office européen des brevets

(11) EP 2 246 099 A2

(12) # EUROPÄISCHE PATENTANMELDUNG

(43) Veröffentlichungstag:
**03.11.2010 Patentblatt 2010/44**

(51) Int Cl.:
***A61Q 5/10*** *(2006.01)* ***A61K 8/55*** *(2006.01)*

(21) Anmeldenummer: **09175701.3**

(22) Anmeldetag: **11.11.2009**

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**
Benannte Erstreckungsstaaten:
**AL BA RS**

(30) Priorität: **25.11.2008 DE 102008058921**

(71) Anmelder: **Henkel AG & Co. KGaA**
**40589 Düsseldorf (DE)**

(72) Erfinder:
- **Goutsis, Konstantin**
**41812, Erkelenz (DE)**
- **Sünger, Georg**
**40547, Düsseldorf (DE)**

(54) **Neue Färbemittel**

(57) Die Erfindung betrifft ein Mittel zur Farbveränderung von keratinhaltigen Fasern, insbesondere menschlichen Haaren, welches neben einer farbgebenden Komponente zusätzlich als amphoteres Tensid einen Phosphorsäureester von ethoxylierten, fettkettenhaltigen Aminen enthält. Im Rahmen einer oxidativen Färbung führt die Verwendung von Mitteln, enthaltend diese amphoteren Tenside zu einer Verbesserung der Egalisierung und zu einer Verbesserung der Waschechtheit.

EP 2 246 099 A2

## Beschreibung

Die Erfindung betrifft ein Mittel zur Farbveränderung von keratinhaltigen Fasern, insbesondere menschlichen Haaren, welches neben einer farbgebenden Komponente zusätzlich als amphoteres Tensid einen Phosphorsäureester von ethoxylierten, fettkettenhaltigen Aminen enthält. Im Rahmen einer oxidativen Färbung führt die Verwendung von Mitteln, enthaltend diese amphoteren Tenside, zu einer Verbesserung der Egalisierung und zu einer Verbesserung der Waschechtheit.

Die Veränderung von Form und Farbe der Haare stellt einen wichtigen Bereich der modernen Kosmetik dar. Dadurch kann das Erscheinungsbild der Haare sowohl aktuellen Modeströmungen als auch den individuellen Wünschen der einzelnen Person angepasst werden. Zur modischen Farbgestaltung von Frisuren oder zur Kaschierung von ergrautem oder gar weißem Haar mit modischen oder natürlichen Farbtönen greift der Verbraucher zu farbverändernden Mitteln.

Zur Bereitstellung farbverändernder kosmetischer Mittel, insbesondere für die Haut oder keratinhaltige Fasern wie beispielsweise menschliche Haare, kennt der Fachmann je nach Anforderungen an die Färbung bzw. Farbveränderung diverse Systeme. Diese Mittel sollen neben der gewünschten Färbeleistung möglichst minimale Schädigungen auf dem Haar hervorrufen und vorzugsweise sogar zusätzliche Pflegeeigenschaften besitzen.

Für permanente, intensive Färbungen mit entsprechenden Echtheitseigenschaften werden sogenannte Oxidationsfärbemittel verwendet. Solche Färbemittel enthalten üblicherweise Oxidationsfarbstoffvorprodukte, sogenannte Entwicklerkomponenten und Kupplerkomponenten. Die Entwicklerkomponenten bilden unter dem Einfluss von Oxidationsmitteln oder von Luftsauerstoff untereinander oder unter Kupplung mit einer oder mehreren Kupplerkomponenten die eigentlichen Farbstoffe aus. Die Oxidationsfärbemittel zeichnen sich durch intensive hervorragende, lang anhaltende Färbeergebnisse aus. Für natürlich wirkende Färbungen muss aber üblicherweise eine Mischung aus einer größeren Zahl von Oxidationsfarbstoffvorprodukten eingesetzt werden, wobei in vielen Fällen weiterhin zusätzlich direktziehende Farbstoffe zur Nuancierung verwendet werden.

Für temporäre Färbungen werden üblicherweise Färbe- oder Tönungsmittel verwendet, die als färbende Komponente sogenannte Direktzieher enthalten. Hierbei handelt es sich um Farbstoffmoleküle, die direkt auf das Substrat aufziehen und keinen oxidativen Prozess zur Ausbildung der Farbe benötigen.

Sollen Substrate aufgehellt oder gar gebleicht werden, werden die das Substrat färbenden Farbstoffe meist oxidativ unter Einsatz von entsprechenden Oxidationsmitteln, wie beispielsweise Wasserstoffperoxid, entfärbt.

Oxidative Haarfärbemittel sind trotz ihrer vorteilhaften Färbeeigenschaften für den Anwender mit Nachteilen behaftet. Selbst wenn oxidative Färbungen deutlich bessere Echtheitseigenschaften als temporäre Färbungen besitzen, so neigen auch oxidative Färbungen zu Farbverlusten gegenüber äußeren Einflüssen. Solche belastenden äußeren Einflüsse sind unter anderem Sonnenlicht, Luftfeuchtigkeit, Chemikalien, wie sie in anderen Haarbehandlungsmitteln oder auch in chloriertem Schwimmbadwasser enthalten sind, insbesondere aber auch die Haarwäsche selbst, bei der nach wiederholter Anwendung häufig ein deutlicher Farbverlust bemerkbar ist. Eine weitere Herauforderung für Färbungen von keratinischen Fasern stellt der Wunsch des Verbrauchers an eine möglichst gleichmäßige Färbung dar. Dies ist insbesondere dann von Interesse, wenn die Haarfaser über ihre Länge einen unterschiedlichen Vorbehandlungsgrad und damit Schädigungsgrad aufweist. Bei der Färbung zur Kaschierung ergrauter Haare ist daher ein einheitliches Färbeergebnis sowohl auf den üblicherweise bereits vorgefärbten Längen der Faser wie auch auf den ungefärbten, nachgewachsenen Haaransatz von großer Bedeutung. Der Fachmann bezeichnet diese Anforderung an das Färbemittel als gutes Egalisiervermögen. Weiterhin führt der Einsatz der Oxidationsmittel zur Ausfärbung beziehungsweise Entwicklung der eigentlichen Färbung zu Schädigungen in der Haarstruktur und auf der Haaroberfläche. Das Haar wird brüchig, seine Elastizität lässt nach, und die Kämmbarkeit nimmt ab. Diese Schädigung nimmt mit der Anwendungsdauer zu. Es ist auch aus Gründen des Anwendungskomforts nahe liegend, dass bei den Benutzern solcher Haarfärbemittel ein Bedürfnis besteht, diese Einwirkungszeit zu verringern. Schließlich benötigen oxidative Färbemittel in der Regel einen basischen pH-Wert zur Ausfärbung, insbesondere zwischen pH 9,0 und pH 10,5. Diese pH-Werte sind notwendig, um eine Öffnung der äußeren Schuppenschicht (Cuticula) zu gewährleisten und eine Penetration der aktiven Spezies (Farbstoffvorprodukte und/oder Wasserstoffperoxid) in das Haar zu ermöglichen. Die Spreizung der äußeren Schuppenschicht führt zu einem unangenehmen Oberflächenempfinden der Haare und damit zu einer verschlechterten Kämmbarkeit im Nass- und Trockenzustand. Es besteht daher ein Bedarf an Färbemitteln, die bei einem geringeren pH-Wert die gewünschte Färbung erzielen, so dass der Verbraucher auf die Verwendung zusätzlicher Nachbehandlungsmittel wie Konditioniermittel möglichst weitestgehend verzichten kann.

Aufgabe der vorliegenden Erfindung ist es daher, die oben genannten Nachteile oxidativer Haarfärbemittel herabzusenken. Die Färbemittel sollen leuchtende, intensive Färbungen des Haars in einer Vielzahl modischer und natürlicher Nuancen ermöglichen. Weiterhin sollen Färbemittel ein sehr gutes Egalisiervermögen und eine verbesserte Grauabdeckung besitzen. Schließlich sollen die erhaltenen Färbungen eine gute Beständigkeit gegenüber äußeren Einflüssen, insbesondere eine gute Lichtechtheit und hervorragende Waschechtheit, besitzen, welche auch nach mehrmaligem Shampoonieren der Haare keine Farbabschwächung oder Farbverschiebung erleiden.

Es wurde überraschend gefunden, dass der Einsatz von speziellen amphoteren Tensiden vom Typ der Phosphorsäureester mit ethoxylierten, fettkettenhaltigen Aminen in Mitteln zur Färbung keratinischer Fasern zu Färbungen von hoher Brillanz und Leuchtvermögen mit sehr guten Waschechtheiten. Ebenso eignen sich solche Mittel hervorragend zur egalisierenden Färbung von bereits vorbehandelten und unbehandelten Haarpartien.

Ein erster Gegenstand der Erfindung ist daher ein Mittel zur Farbveränderung keratinischer Fasern, enthaltend in einem kosmetischen Träger mindestens eine farbgebende Komponente, dadurch gekennzeichnet, dass es zusätzlich mindestens eine Verbindung gemäß Formel (I) und/oder eines ihrer physiologisch verträglichen Salze enthält,

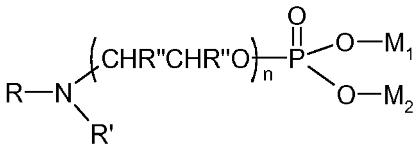

(I),

worin

| | |
|---|---|
| R | für eine $C_8$-$C_{30}$-Alkylgruppe, welche bis zu 4 Doppelbindungen enthalten kann, steht, |
| R' | für ein Wasserstoffatom, eine $C_1$-$C_{30}$-Alkylkette, eine $C_2$-$C_{30}$-Hydroxyalkylkette oder eine Gruppe $(CH_2CH_2O)_m$-Y steht, wobei Y für ein Wasserstoffatom, einen Sulfatester $S(=O)_2OM_3$ oder einen Phosphatester $P(=O)(OM_3)(OM_4)$ steht und m für eine ganze Zahl von 1 bis 50 steht, |
| R" | unabhängig voneinander für ein Wasserstoffatom oder eine $C_1$-$C_4$-Alkylkette steht, |
| n | für eine ganze Zahl von 1 bis 50 steht, |
| und $M_1$, $M_2$ | sowie gegebenenfalls $M_3$ und $M_4$ jeweils unabhängig voneinander für ein Wasserstoffatom, ein Metallatom oder eine gegebenenfalls alkylierte Ammonium-Einheit steht. |

Unter keratinischen Fasern sind Wolle, Pelze, Federn und insbesondere menschliche Haare zu verstehen. Die erfindungsgemäßen Färbemittel können prinzipiell aber auch zum Färben anderer Naturfasern, wie z. B. Baumwolle, Jute, Sisal, Leinen oder Seide, modifizierter Naturfasern, wie beispielsweise Regeneratcellulose, Nitro-, Alkyl- oder Hydroxyalkyl- oder Acetylcellulose verwendet werden.

Die erfindungsgemäßen Mittel enthalten die farbgebenden Komponenten und die Verbindungen der Formel (I) in einem kosmetischen Träger, bevorzugt in einem geeigneten wässrigen, alkoholischen oder wässrig-alkoholischen Träger. Zum Zwecke der Haarfärbung sind solche Träger beispielsweise Cremes, Emulsionen, Gele oder auch tensidhaltige schäumende Lösungen, wie beispielsweise Shampoos, Schaumaerosole oder andere Zubereitungen, die für die Anwendung auf dem Haar geeignet sind. Unter wässrig-alkoholischen Lösungen sind im Sinne der vorliegenden Erfindung wässrige Lösungen enthaltend 3 bis 70 Gew.-% eines $C_1$-$C_4$-Alkohols, insbesondere Ethanol bzw. Isopropanol, zu verstehen. Die erfindungsgemäßen Mittel können zusätzlich weitere organische Lösemittel, wie beispielsweise Methoxybutanol, Benzylalkohol, Ethyldiglykol oder 1,2-Propylenglykol, enthalten. Bevorzugt sind dabei alle wasserlöslichen organischen Lösemittel. Ein wässriger Träger bedeutet im Rahmen der Erfindung, dass das anwendungsbereite Mittel zu wenigstens 30 Gew.-%, insbesondere 50 Gew.-%, Wasser enthält.

In den erfindungsgemäßen amphoteren Tensiden gemäß Formel (I) steht R für eine $C_8$-$C_{30}$-Alkylgruppe, welche bis zu 4 Doppelbindungen enthalten kann. Bevorzugte Alkylgruppen leiten sich dabei von Reduktionsprodukten natürlicher und/oder synthetischer Fettsäuren ab, bevorzugt mit einer Kettenlänge von 8 bis 22 C-Atomen. Besonders bevorzugte Alkylreste sind daher Octyl, Decyl, Dodecyl (Lauryl), Tetradecyl (Myristyl), Hexadecyl (Cetyl), 9-Hexadecenyl (Palmitoleyl), Octadecyl (Stearyl), Isostearyl, 9-(Z)-Octadecenyl (Oleyl), 9-(E)-Octadecenyl (Elaidyl), 6-(Z)-Octadecenyl (Petroselinyl), Eicosyl (Arachyl), Docosanyl (Behenyl), 13-(Z)-Docosenyl (Erucyl), 13-(E)-Docosenyl (Brassidyl) sowie technische Gemische von Alkylresten mit unterschiedlicher Kettenlänge und Doppelbindungsanzahl. Solche Gemische fallen beispielsweise als natürliche Gemische aus den Fettsäuren pflanzlichen oder tierischen Ursprungs an, wie insbesondere Cocosalkylreste mit Alkylkettenlängen von 8 bis 18 C-Atomen mit einem hohen Anteil an $C_{12}$/$C_{14}$-Alkylresten oder Talgalkylreste mit Alkylkettenlängen von 14 bis 18 C-Atomen mit einem hohen Anteil an $C_{14}$/$C_{16}$/$C_{18}$-Alkylresten.

Der Rest R' des erfindungsgemäßen Tensids gemäß Formel (I) steht für ein Wasserstoffatom, eine $C_1$-$C_{30}$-Alkylkette, eine $C_2$-$C_{30}$-Hydroxyalkylkette oder eine Gruppe $(CH_2CH_2O)_m$-Y steht, wobei Y für ein Wasserstoffatom, einen Sulfatester $S(=O)_2OM_3$ oder einen Phosphatester $P(=O)(OM_3)(OM_4)$, worin m für eine ganze Zahl von 1 bis 50 steht.

Bevorzugte erfindungsgemäße Verbindungen gemäß Formel (I) sind dadurch gekennzeichnet, dass in Formel (I) R' für eine Gruppe $(CH_2CH_2O)_m$-Y steht, wobei Y für einen Sulfatester $S(=O)_2OM_3$ oder einen Phosphatester $P(=O)(OM_3)(OM_4)$ und m für eine ganze Zahl von 1 bis 50 steht. Bevorzugte Verbindungen sind dadurch gekennzeichnet, dass m für eine Zahl größer als 1 steht.

Der Rest R" steht für ein Wasserstoffatom oder eine $C_1$-$C_4$-Alkylkette. Besonders bevorzugt wird der Rest R" ausgewählt aus Wasserstoff oder einem Methylrest, wobei solche Verbindungen gemäß Formel (I) wiederum bevorzugt

sind, worin entweder einer der beiden Reste R" für Wasserstoff und der andere für eine Methylgruppe steht oder beide Reste R" für ein Wasserstoffatom stehen.

Die Reste $M_1$, $M_2$ sowie gegebenenfalls $M_3$ und $M_4$ stehen vorzugsweise für ein Wasserstoffatom, ein Metallatom oder eine gegebenenfalls alkylierte Ammonium-Einheit steht. Wenn $M_1$, $M_2$ sowie gegebenenfalls $M_3$ und $M_4$ für ein Metallatom oder eine Ammonium-Einheit stehen, ist selbstverständlich, dass die Bindung zum benachbarten Sauerstoffatom eine ionische Bindung darstellt, so dass der Sauerstoff eine negative Ladung trägt, während das Metallatom oder Ammonium-Einheit als positiv geladenes Ion vorliegen. Bevorzugte Metallionen leiten sich dabei von Alkalimetallen, Erdalkalimetallen, Aluminium oder Zink ab. Ganz besonders bevorzugte Metallionen sind dabei abgeleitet von Natrium, Kalium und Magnesium. Es ist selbstverständlich, dass mehrwertige Kationen entsprechend ihrer stöchiometrischen Ladung einzusetzen sind, wie beispielsweise ½ $Mg^{2+}$ oder ⅓ $Al^{3+}$. Die Ammonium-Ionen können mit bis zu 4 gegebenenfalls hydroxylierten Alkylgruppen alkyliert sein. Bevorzugte Alkylreste sind dabei Methyl, Ethyl, Propyl, Isopropyl oder Butyl, sowie insbesondere 2-Hydroxyethyl.

Es hat sich als erfindungsgemäß besonders vorteilhaft herausgestellt, wenn in Formel (I) R für eine $C_{10}$-$C_{22}$-Alkylgruppe, welche bis zu 4 Doppelbindungen enthalten kann, steht. Erfindungsgemäß besonders bevorzugte Verbindungen sind dadurch gekennzeichnet, dass der Rest R ausgewählt ist aus Lauryl, Cetyl, Stearyl, Oleyl sowie Cocosalkyl (Gemisch aus $C_8$ bis $C_{18}$), Talgalkyl (Gemisch aus $C_{14}/C_{16}/C_{18}$) sowie Cetearyl (Gemisch aus $C_{16}/C_{18}$).

Die Zahl n in Formel (I) gibt die Anzahl der Alkoxy-Einheiten der Phospatester-Kette an. Dabei steht n für eine Zahl von 1 bis 50. Es kann erfindungsgemäß vorteilhaft sein, wenn n für eine Zahl zwischen 1 und 15, bevorzugt zwischen 2 und 10, ganz besonders bevorzugt zwischen 3 und 8 steht. In einer weiteren Ausführungsform steht n bevorzugt für eine Zahl zwischen 10 und 35, bevorzugt zwischen 12 und 30, ganz besonders bevorzugt zwischen 15 und 25.

Besonders vorteilhafte Mittel sind dadurch gekennzeichnet, dass das Tensid eine Verbindung gemäß Formel (I) darstellt, worin R" für ein Wasserstoffatom oder eine Methylgruppe steht und n für eine ganze Zahl von 1 bis 25 steht.

Eine besonders bevorzugte Ausführungsform des erfindungsgemäßen Mittels ist dadurch gekennzeichnet, dass in Formel (I) der Rest R' für eine ethoxylierte Phosphatester Gruppe $(CH_2CH_2O)_m$-P(=O)$(OM_3)(OM_4)$ steht und m für eine ganze Zahl von 1 bis 25 steht.

Es ist besonders bevorzugt, wenn das amphotere Tensid durch eine Verbindung gemäß Formel (I) wiedergegeben ist, in der der Rest R' für eine ethoxylierte Phosphatester Gruppe $(CH_2CH_2O)_m$-P(=O)$(OM_3)(OM_4)$ steht, der Rest R" für ein Wasserstoffatom steht und n und m jeweils unabhängig voneinander für eine ganze Zahl von 1 bis 25 stehen.

Solche erfindungsgemäß besonders bevorzugten Verbindungen sind durch die Formel (Ia) gegeben,

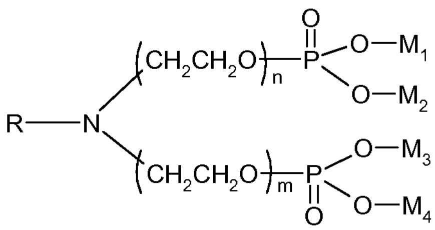

(Ia),

worin n und m jeweils unabhängig voneinander für eine Zahl von 1 bis 25. Bevorzugte Verbindungen zeichnen sich dadurch aus, dass n und m jeweils unabhängig voneinander für eine Zahl zwischen 1 und 15, bevorzugt zwischen 2 und 10, ganz besonders bevorzugt zwischen 2 und 6 steht. Ganz besonders bevorzugte Verbindungen sind **dadurch gekennzeichnet, dass** die Summe aus n und m eine Zahl zwischen 8 und 12, bevorzugt 9 bis 11 ist.

Bevorzugt steht der Rest R in einer Verbindung gemäß Formel (Ia) für eine $C_8$-$C_{30}$-Alkylgruppe, welche bis zu 4 Doppelbindungen enthalten kann. Ganz besonders bevorzugte Alkylgruppen leiten sich dabei von Reduktionsprodukten natürlicher und/oder synthetischer Fettsäuren ab, bevorzugt mit einer Kettenlänge von 8 bis 22 C-Atomen. Besonders bevorzugte Alkylreste sind daher Octyl, Decyl, Dodecyl, Tetradecyl, Hexadecyl, 9-Hexadecenyl, Octadecyl, Isostearyl, 9-(Z)-Octadecenyl, 9-(E)-Octadecenyl, 6-(Z)-Octadecenyl, Eicosyl, Docosanyl, 13-(Z)-Docosenyl, 13-(E)-Docosenyl sowie technische Gemische von Alkylresten mit unterschiedlicher Kettenlänge und Doppelbindungsanzahl. Solche Gemische fallen beispielsweise als natürliche Gemische aus den Fettsäuren pflanzlichen Ursprungs an, wie insbesondere Cocosalkyl-Reste mit Alkylkettenlängen von 8 bis 18 C-Atomen mit einem hohen Anteil an $C_{12}/C_{14}$-Alkylresten. Ganz besonders vorteilhafte Verbindungen sind **dadurch gekennzeichnet, dass** R für einen Talgalkylrest, d. h. ein Gemisch aus überwiegend $C_{14}/C_{16}/C_{18}$-Alkylresten steht

Erfindungsgemäß besonders bevorzugte Verbindungen sind **dadurch gekennzeichnet, dass** in Formel (I) R" für ein Wasserstoffatom, R für einen Talgalkylrest und die Summe aus n und m eine ganze Zahl zwischen 8 bis 12 steht.

Erfindungsgemäß bevorzugte Verbindungen gemäß Formel (Ia), worin die Summe aus n und m eine Zahl zwischen 8 und 12 und R für einen Talgalkylrest steht, werden beispielsweise von der Firma Bozzetto unter dem

Handelsnamen Produkt T4835 vertrieben.

Bevorzugt enthält das Mittel das erfindungsgemäße amphotere Tensid in einer Menge von 0,01 bis 10 Gew.-%, bevorzugt von 0,05 bis 8 Gew.-%, insbesondere bevorzugt 0,1 bis 5 Gew.-%, jeweils bezogen auf das Gesamtgewicht des anwendungsbereiten Mittels.

Das erfindungsgemäße Mittel enthält mindestens eine farbgebende Komponente. Bevorzugte Verbindungen zeichnen sich dadurch aus, dass sie als farbgebende Komponente mindestens eine Verbindung enthält, die ausgewählt ist aus (a) Oxidationsfarbstoffvorprodukten und/oder (b) naturanalogen Farbstoffen und/oder (c) direktziehenden Farbstoffen.

In einer bevorzugten Ausführungsform enthält das Mittel als farbgebende Komponente mindestens ein Oxidationsfarbstoffvorprodukt.

Oxidationsfarbstoffvorprodukte können aufgrund ihres Reaktionsverhaltens in zwei Kategorien eingeteilt werden, sogenannte Entwicklerkomponenten und Kupplerkomponenten. Entwicklerkomponenten können mit sich selbst den eigentlichen Farbstoff ausbilden. Sie können daher als alleinige, farbverändernde Verbindungen im erfindungsgemäßen Mittel enthalten sein. In einer bevorzugten Ausführungsform enthalten die erfindungsgemäßen Mittel mindestens ein Oxidationsfarbstoffvorprodukt vom Entwicklertyp und/oder Kupplertyp. Vorzugsweise enthalten die erfindungsgemäßen Färbemittel mindestens ein Oxidationsfarbstoffvorprodukt vom Entwicklertyp und mindestens ein Oxidationsfarbstoffvorprodukt vom Kupplertyp.

Die Entwickler- und Kupplerkomponenten werden üblicherweise in freier Form eingesetzt. Bei Substanzen mit Aminogruppen kann es aber bevorzugt sein, sie in Salzform, insbesondere in Form der Hydrochloride und Hydrobromide oder der Sulfate einzusetzen.

Nachfolgend werden erfindungsgemäße Entwicklerkomponenten eingehender vorgestellt. Besonders bevorzugt sind p-Phenylendiaminderivate. Besonders bevorzugte p-Phenylendiamine werden ausgewählt aus einer oder mehrerer Verbindungen der Gruppe, die gebildet wird, aus 1,4-Diaminobenzol (p-Phenylendiamin), 1,4-Diamino-2-methylbenzol (p-Toluylendiamin), 1,4-Diamino-2-chlorbenzol (2-Chlor-p-phenylendiamin), 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-3-methyl-(N,N-diethyl)-anilin, N,N-Bis-(2-hydroxyethyl)-p-phenylendiamin, 4-N,N-Bis-(2-hydroxyethyl)amino-2-methylanilin, 4-N,N-Bis-(2-hydroxyethyl)-amino-2-chloranilin, 2-(2-Hydroxyethyl)-p-phenylendiamin, 2-(1,2-Dihydroxyethyl)-p-phenylendiamin, 2-Fluor-p-phenylendiamin, 2-Isopropyl-p-phenylendiamin, N-(2-Hydroxypropyl)-p-phenylendiamin, 2-Hydroxymethyl-p-phenylendiamin, N,N-Dimethyl-3-methyl-p-phenylendiamin, N-Ethyl-N-2-hydroxyethyl-p-phenylendiamin, N-(2,3-Dihydroxypropyl)-p-phenylendiamin, N-(4'-Aminophenyl)-p-phenylendiamin, N-Phenyl-p-phenylendiamin, 2-(2-Hydroxyethyloxy)-p-phenylendiamin, 2-Methoxymethyl-p-phenylendiamin, 2-(2-Acetylaminoethyloxy)-p-phenylendiamin, N-(2-Methoxyethyl)-p-phenylendiamin, N-(4-Amino-3-methylphenyl)-N-[3-(1H-imidazol-1-yl)propyl]amin, 5,8-Diaminobenzo-1,4-dioxan sowie ihren physiologisch verträglichen Salzen. Erfindungsgemäß ganz besonders bevorzugte p-Phenylendiaminderivate sind ausgewählt aus p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, N-(4-Amino-3-methylphenyl)-N-[3-(1H-imidazol-1-yl)propyl]amin, 2-Methoxymethyl-p-phenylendiamin sowie deren physiologisch verträglichen Salzen.

Es kann erfindungsgemäß weiterhin bevorzugt sein, als Entwicklerkomponente Verbindungen einzusetzen, die mindestens zwei aromatische Kerne enthalten, die mit Amino- und/oder Hydroxylgruppen substituiert sind. Bevorzugte zweikernige Entwicklerkomponenten werden aus mindestens einer der folgenden Verbindungen ausgewählt: N,N'-Bis-(2-hydroxyethyl)-N,N'-bis-(4'-aminophenyl)-1,3-diaminopropan-2-ol, N,N'-Bis-(2-hydroxyethyl)-N,N'-bis-(4'-aminophenyl)ethylendiamin, N,N'-Bis-(4'-aminophenyl)tetramethylendiamin, N,N'-Bis-(2-hydroxyethyl)-N,N'-bis-(4'-aminophenyl)-tetramethylendiamin, N,N'-Bis-(4-(methylamino)phenyl)tetramethylendiamin, N,N'-Diethyl-N,N'-bis-(4'-amino-3'-methylphenyl)ethylendiamin, Bis-(2-hydroxy-5-aminophenyl)methan, N,N'-Bis-(4'-aminophenyl)-1,4-diazacycloheptan, N,N'-Bis-(2-hydroxy-5-aminobenzyl)piperazin, N-(4'-Aminophenyl)-p-phenylendiamin und 1,10-Bis-(2',5'-diaminophenyl)-1,4,7,10-tetraoxadecan sowie ihre physiologisch verträglichen Salze. Ganz besonders bevorzugte zweikernige Entwicklerkomponenten werden ausgewählt unter N,N'-Bis-(2-hydroxyethyl)-N,N'-bis-(4-aminophenyl)-1,3-diaminopropan-2-ol, Bis-(2-hydroxy-5-aminophenyl)methan, 1,3-Bis-(2,5-diaminophenoxy)propan-2-ol, N,N'-Bis-(4-aminophenyl)-1,4-diazacycloheptan, 1,10-Bis-(2,5-diaminophenyl)-1,4,7,10-tetraoxadecan oder eines deren physiologisch verträglichen Salze.

Weiterhin kann es erfindungsgemäß bevorzugt sein, als Entwicklerkomponente ein p-Aminophenolderivat oder eines seiner physiologisch verträglichen Salze einzusetzen. Bevorzugte p-Aminophenole sind insbesondere p-Aminophenol, N-Methyl-p-aminophenol, 4-Amino-3-methylphenol, 4-Amino-3-fluorphenol, 2-Hydroxymethylamino-4-aminophenol, 4-Amino-3-hydroxymethylphenol, 4-Amino-2-(2-hydroxyethoxy)-phenol, 4-Amino-2-methylphenol, 4-Amino-2-hydroxymethylphenol, 4-Amino-2-methoxymethyl-phenol, 4-Amino-2-aminomethylphenol, 4-Amino-2-(2-hydroxyethyl-aminomethyl)-phenol, 4-Amino-2-(1,2-dihydroxyethyl)-phenol, 4-Amino-2-fluorphenol, 4-Amino-2-chlorphenol, 4-Amino-2,6-dichlorphenol, 4-Amino-2-(diethylaminomethyl)-phenol sowie ihre physiologisch verträglichen Salze. Ganz besonders bevorzugte Verbindungen sind p-Aminophenol, 4-Amino-3-methylphenol, 4-Amino-2-aminomethylphenol, 4-Amino-2-(1,2-dihydroxyethyl)-phenol und 4-Amino-2-(diethylaminomethyl)phenol.

Ferner kann die Entwicklerkomponente ausgewählt sein aus o-Aminophenol und seinen Derivaten, wie 2-Amino-4-methylphenol, 2-Amino-5-methylphenol oder 2-Amino-4-chlorphenol.

Weiterhin kann die Entwicklerkomponente ausgewählt sein aus heterocyclischen Entwicklerkomponenten, wie beispielsweise aus Pyrimidinderivaten, Pyrazolderivaten, Pyrazolopyrimidin-Derivaten bzw. ihren physiologisch verträglichen Salzen. Bevorzugte Pyrimidin-Derivate sind insbesondere die Verbindungen 2,4,5,6-Tetraaminopyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin und 2,5,6-Triaminopyrimidin. Bevorzugte Pyrazol-Derivate sind insbesondere die Verbindungen, die ausgewählt werden unter 4,5-Diamino-1-methylpyrazol, 4,5-Diamino-1-(2-hydroxyethyl)pyrazol, 3,4-Diaminopyrazol, 4,5-Diamino-1-(4'-chlorbenzyl)pyrazol, 4,5-Diamino-1,3-dimethylpyrazol, 4,5-Diamino-3-methyl-1-phenylpyrazol, 4,5-Diamino-1-methyl-3-phenylpyrazol, 4-Amino-1,3-dimethyl-5-hydrazinopyrazol, 1-Benzyl-4,5-diamino-3-methylpyrazol, 4,5-Diamino-3-t-butyl-1-methylpyrazol, 4,5-Diamino-1-t-butyl-3-methylpyrazol, 4,5-Diamino-1-(2-hydroxyethyl)-3-methylpyrazol, 4,5-Diamino-1-ethyl-3-methylpyrazol, 4,5-Diamino-1-ethyl-3-(4-methoxyphenyl)pyrazol, 4,5-Diamino-1-ethyl-3-hydroxymethylpyrazol, 4,5-Diamino-3-hydroxymethyl-1-methylpyrazol, 4,5-Diamino-3-hydroxymethyl-1-isopropylpyrazol, 4,5-Diamino-3-methyl-1-isopropylpyrazol, 4-Amino-5-(2-aminoethyl)amino-1,3-dimethylpyrazol, sowie deren physiologisch verträglichen Salze, insbesondere jedoch 4,5-Diamino-1-(2-hydroxyethyl)pyrazol. Unter den Pyrazolo[1,5-a]pyrimidinen kann man insbesondere nennen: Pyrazolo[1,5-a]pyrimidin-3,7-diamin; 2,5-Dimethyl-pyrazolo[1,5-a]pyrimidin-3,7-diamin; Pyrazolo[1,5-a]pyrimidin-3,5-diamin; 2,7-Dimethyl-pyrazolo[1,5-a]pyrimidin-3,5-diamin; 3-Aminopyrazolo[1,5-a]pyrimidin-7-ol; 3-Aminopyrazolo[1,5-a]pyrimidin-5-ol; 2-(3-Aminopyrazolo[1,5-a]pyrimidin-7-ylamino)ethanol; 2-(7-Aminopyrazolo[1,5-a]pyrimidin-3-ylamino)ethanol; 2-[(3-Aminopyrazolo[1,5-a]pyrimidin-7-yl)-(2-hydroxyethyl)amino]ethanol; 2-[(7-Aminopyrazolo[1,5-a]pyrimidin-3-yl)-(2-hydroxy-ethyl)-amino]ethanol; 5,6-Dimethylpyrazolo[1,5-a]pyrimidin-3,7-diamin; 2,6-Dimethylpyrazolo[1,5-a]pyrimidin-3,7-diamin; 3-Amino-7-dimethylamino-2,5-dimethylpyrazolo[1,5-a]pyrimidin sowie ihre physiologisch verträglichen Salze und ihre tautomeren Formen, wenn ein tautomeres Gleichgewicht vorhanden ist.

Besonders bevorzugte Entwicklerkomponenten werden ausgewählt aus mindestens einer Verbindung aus der Gruppe, die gebildet wird aus p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, 2-Methoxymethyl-p-phenylendiamin, N-(4-Amino-3-methylphenyl)-N-[3-(1H-imidazol-1-yl)propyl]amin, N,N'-Bis-(2-hydroxyethyl)-N,N'-bis-(4-aminophenyl)-1,3-diaminopropan-2-ol, Bis-(2-hydroxy-5-aminophenyl)methan, 1,3-Bis-(2,5-diaminophenoxy)propan-2-ol, N,N'-Bis-(4-aminophenyl)-1,4-diazacycloheptan, 1,10-Bis-(2,5-diaminophenyl)-1,4,7,10-tetraoxadecan, p-Aminophenol, 4-Amino-3-methylphenol, 4-Amino-2-aminomethylphenol, 4-Amino-2-(1,2-dihydroxyethyl)phenol und 4-Amino-2-(diethylaminomethyl)phenol, 4,5-Diamino-1-(2-hydroxyethyl)pyrazol, 2,4,5,6-Tetraaminopyrimidin, 4-Hydroxy-2,5,6-triaminopyrimidin, 2-Hydroxy-4,5,6-triaminopyrimidin, sowie deren physiologisch verträglichen Salzen. Ganz besonders bevorzugte Entwicklerkomponenten sind dabei p-Toluylendiamin, 2-(2-Hydroxyethyl)-p-phenylendiamin, 2-Methoxymethyl-p-phenylendiamin, N-(4-Amino-3-methylphenyl)-N-[3-(1H-imidazol-1-yl)propyl]-amin, und/oder 4,5-Diamino-1-(2-hydroxyethyl)pyrazol sowie deren physiologisch verträglichen Salze.

Die Entwicklerkomponenten werden bevorzugt in einer Menge von 0,005 bis 20 Gew.-%, vorzugsweise 0,1 bis 5 Gew.-%, jeweils bezogen auf das anwendungsbereite Oxidationsfärbemittel, verwendet.

Kupplerkomponenten bilden im Rahmen der oxidativen Färbung allein keine signifikante Färbung aus, sondern benötigen stets die Gegenwart von Entwicklerkomponenten. Daher ist es erfindungsgemäß bevorzugt, dass bei Verwendung mindestens einer Kupplerkomponente zusätzlich mindestens eine Entwicklerkomponente zum Einsatz kommt.

Erfindungsgemäße Kupplerkomponenten werden bevorzugt als mindestens eine Verbindung aus einer der folgenden Klassen ausgewählt:

3-Aminophenol (m-Aminophenol) und/oder dessen Derivate, 3-Aminoanilin (m-Diaminobenzol) und/oder dessen Derivate, 2-Aminoanilin (1,2-Diaminobenzol; o-Diaminobenzol) und/oder dessen Derivate, 2-Aminophenol (o-Aminophenol) und/oder dessen Derivate, Naphthalinderivate mit mindestens einer Hydroxygruppe, Di- beziehungsweise Trihydroxybenzol und/oder deren Derivate, Pyridinderivate, Pyrimidinderivate, Monohydroxyindol-Derivate und/oder Monoaminoindol-Derivate, Monohydroxyindolin-Derivate und/oder Monoaminoindolin-Derivate, Pyrazolonderivate (wie 1-Phenyl-3-methylpyrazol-5-on), Morpholinderivate (wie 6-Hydroxybenzomorpholin oder 6-Aminobenzomorpholin), Chinoxalinderivate (wie 6-Methyl-1,2,3,4-tetrahydrochinoxalin) sowie Gemische aus zwei oder mehreren Verbindungen aus einer oder mehreren dieser Klassen sind im Rahmen dieser Ausführungsform ebenso erfindungsgemäß.

Bevorzugte 3-Aminophenol-Kupplerkomponenten werden ausgewählt aus mindestens einer Verbindung aus der Gruppe, die gebildet wird aus 3-Aminophenol, 5-Amino-2-methylphenol, N-Cyclopentyl-3-aminophenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)amino-2-methylphenol, 3-Diethylaminophenol, N-Cyclopentyl-3-aminophenol, 1,3-Dihydroxy-5-(methylamino)benzol, 3-Ethylamino-4-methylphenol, 2,4-Dichlor-3-aminophenol und deren physiologisch verträglichen Salzen.

Bevorzugte 3-Diaminobenzol-Kupplerkomponenten werden ausgewählt aus mindestens einer Verbindung aus der Gruppe, die gebildet wird aus 3-Aminoanilin (m-Phenylendiamin), 2-(2,4-Diaminophenoxy)ethanol, 1,3-Bis(2,4-diaminophenoxy)propan, 1-Methoxy-2-amino-4-(2'-hydroxyethylamino)benzol, 1,3-Bis(2,4-diaminophenyl)propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2'-hydroxyethyl)aminobenzol und deren physiologisch verträglichen Salzen.

Bevorzugte 1,2-Diaminobenzol-Kupplerkomponenten werden ausgewählt aus mindestens einer Verbindung aus der Gruppe, die gebildet wird aus 3,4-Diaminobenzoesäure und 2,3-Diamino-1-methylbenzol und deren physiologisch verträglichen Salzen.

Bevorzugte Di- beziehungsweise Trihydroxybenzole und deren Derivate werden ausgewählt aus mindestens einer Verbindung der Gruppe, die gebildet wird aus Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol.

Bevorzugte Pyridinderivate werden ausgewählt aus mindestens einer Verbindung der Gruppe, die gebildet wird aus 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin, 3,5-Diamino-2,6-dimethoxypyridin, 3,4-Diaminopyridin, 2-(2-Methoxyethyl)amino-3-amino-6-methoxypyridin, 2-(4'-Methoxyphenyl)amino-3-aminopyridin, und deren physiologisch verträglichen Salzen.

Bevorzugte Naphthalinderivate mit mindestens einer Hydroxygruppe werden ausgewählt aus mindestens einer Verbindung der Gruppe, die gebildet wird aus 1-Naphthol, 2-Methyl-1-naphthol, 2-Hydroxymethyl-1-naphthol, 2-Hydroxyethyl-1-naphthol, 1,3-Dihydroxynaphthalin, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin.

Bevorzugte Indolderivate werden ausgewählt aus mindestens einer Verbindung der Gruppe, die gebildet wird aus 4-Hydroxyindol, 6-Hydroxyindol und 7-Hydroxyindol und den physiologisch verträglichen Salzen der vorgenannten Verbindungen.

Bevorzugte Indolinderivate werden ausgewählt aus mindestens einer Verbindung der Gruppe, die gebildet wird aus 4-Hydroxyindolin, 6-Hydroxyindolin und 7-Hydroxyindolin und deren physiologisch verträglichen Salzen.

Bevorzugte Pyrimidinderivate werden ausgewählt aus mindestens einer Verbindung der Gruppe, die gebildet wird aus 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin und deren physiologisch verträglichen Salzen.

Erfindungsgemäß besonders bevorzugte Kupplerkomponenten werden ausgewählt unter 3-Aminophenol, 5-Amino-2-methylphenol, 3-Amino-2-chlor-6-methylphenol, 2-Hydroxy-4-aminophenoxyethanol, 5-Amino-4-chlor-2-methylphenol, 5-(2-Hydroxyethyl)-amino-2-methylphenol, 2,4-Dichlor-3-aminophenol, 2-Aminophenol, 3-Phenylendiamin, 2-(2,4-Diaminophenoxy)ethanol, 1,3-Bis(2,4-diaminophenoxy)propan, 1-Methoxy-2-amino-4-(2-hydroxyethylamino)benzol, 1,3-Bis(2,4-diaminophenyl)propan, 2,6-Bis(2'-hydroxyethylamino)-1-methylbenzol, 2-({3-[(2-Hydroxyethyl)-amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2-hydroxyethyl)aminobenzol, Resorcin, 2-Methylresorcin, 4-Chlorresorcin, 1,2,4-Trihydroxybenzol, 2-Amino-3-hydroxypyridin, 3-Amino-2-methylamino-6-methoxypyridin, 2,6-Dihydroxy-3,4-dimethylpyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydroxyindol, 7-Hydroxyindol, 4-Hydroxyindolin, 6-Hydroxyindolin, 7-Hydroxyindolin oder Gemischen dieser Verbindungen oder deren physiologisch verträglichen Salzen. Ganz besonders bevorzugt ist dabei Resorcin, 2-Methylresorcin, 5-Amino-2-methylphenol, 3-Aminophenol, 2-(2,4-Diaminophenoxy)ethanol, 1,3-Bis(2,4-diaminophenoxy)-propan, 1-Methoxy-2-amino-4-(2'-hydroxyethylamino)benzol, 2-Amino-3-hydroxypyridin und 1-Naphthol sowie eines deren physiologisch verträglichen Salze.

Die Kupplerkomponenten werden bevorzugt in einer Menge von 0,005 bis 20 Gew.-%, vorzugsweise 0,1 bis 5 Gew.-%, jeweils bezogen auf das anwendungsbereite Oxidationsfärbemittel, verwendet.

Im Rahmen der vorliegenden Erfindung sind folgende Kombinationen aus Oxidationsfarbstoffvorprodukten vom Entwicklertyp und vom Kupplertyp besonders bevorzugt Mit den als Kombination genannten Oxidationsfarbstoffvorprodukten können jedoch auch noch weitere Farbstoffvorprodukte kombiniert werden:

p-Toluylendiamin / Resorcin; p-Toluylendiamin / 2-Methylresorcin; p-Toluylendiamin / 5-Amino-2-methylphenol; p-Toluylendiamin / 3-Aminophenol; p-Toluylendiamin / 2-(2,4-Diaminophenoxy)-ethanol; p-Toluylendiamin / 1,3-Bis(2,4-diaminophenoxy)propan; p-Toluylendiamin / 1-Methoxy-2-amino-4-(2-hydroxyethylamino)benzol; p-Toluylendiamin / 2-Amino-3-hydroxypyridin; p-Toluylendiamin / 1-Naphthol; 2-(2-Hydroxyethyl)-p-phenylendiamin / Resorcin; 2-(2-Hydroxyethyl)-p-phenylendiamin / 2-Methylresorcin; 2-(2-Hydroxyethyl)-p-phenylendiamin / 5-Amino-2-methylphenol; 2-(2-Hydroxyethyl)-p-phenylendiamin / 3-Aminophenol; 2-(2-Hydroxyethyl)-p-phenylendiamin / 2-(2,4-Diaminophenoxy)ethanol; 2-(2-Hydroxyethyl)-p-phenylendiamin / 1,3-Bis(2,4-diaminophenoxy)propan; 2-(2-Hydroxyethyl)-p-phenylendiamin / 1-Methoxy-2-amino-4-(2-hydroxyethylamino)benzol; 2-(2-Hydroxyethyl)-p-phenylendiamin / 2-Amino-3-hydroxypyridin; 2-(2-Hydroxyethyl)-p-phenylendiamin / 1-Naphthol; 2-Methoxymethyl-p-phenylendiamin / Resorcin; 2-Methoxymethyl-p-phenylendiamin / 2-Methylresorcin; 2-Methoxymethyl-p-phenylendiamin / 5-Amino-2-methylphenol; 2-Methoxymethyl-p-phenylendiamin / 3-Aminophenol; 2-Methoxymethyl-p-phenylendiamin / 2-(2,4-Diaminophenoxy)ethanol; 2-Methoxymethyl-p-phenylendiamin / 1,3-Bis-(2,4-diaminophenoxy)propan; 2-Methoxymethyl-p-phenylendiamin / 1-Methoxy-2-amino-4-(2-hydroxyethylamino)benzol; 2-Methoxymethyl-p-phenylendiamin / 2-Amino-3-hydroxypyridin; 2-Methoxymethyl-p-phenylendiamin / 1-Naphthol; N-(4-Amino-3-methylphenyl)-N-[3-(1H-imidazol-1-yl)propyl]amin / Resorcin; N-(4-Amino-3-methylphenyl)-N-[3-(1H-imidazol-1-yl)propyl]amin / 2-Methylresorcin; N-(4-Amino-3-methylphenyl)-N-[3-(1H-imidazol-1-yl)propyl]amin / 5-Amino-2-methylphenol; N-(4-Amino-3-methylphenyl)-N-[3-(1H-imidazol-1-yl)propyl]amin / 3-Aminophenol; N-(4-Amino-3-methylphenyl)-N-[3-(1H-imidazol-1-yl)propyl]amin / 2-(2,4-Diaminophenoxy)ethanol; N-(4-Amino-3-methylphenyl)-N-[3-(1H-imidazol-1-yl)propyl]amin / 1,3-Bis(2,4-diaminophenoxy)-propan; N-(4-Amino-3-methylphenyl)-N-[3-(1H-imidazol-1-yl)propyl]amin / 1-Methoxy-2-amino-4-(2-hydroxyethylamino)benzol; N-(4-Amino-3-methylphenyl)-N-[3-(1H-imidazol-1-yl)propyl]amin / 2-Amino-3-hydroxypyridin; N-(4-Amino-3-methylphenyl)-N-[3-(1H-imidazol-1-yl)propyl]amin / 1-Naphthol; 4,5-Diamino-1-(2-hydroxyethyl)pyrazol / Resorcin; 4,5-Diamino-1-(2-hydroxyethyl)-pyrazol / 2-Methylresorcin; 4,5-Diamino-1-(2-hydroxyethyl)pyrazol / 5-Amino-2-methylphenol; 4,5-Diamino-1-(2-hydroxyethyl)pyrazol / 3-Aminophenol; 4,5-Diamino-1-(2-hydroxyethyl)pyrazol / 2-(2,4-Diaminophenoxy)ethanol; 4,5-Diamino-1-(2-hydroxyethyl)pyrazol / 1,3-Bis(2,4-diaminophenoxy)propan; 4,5-Diamino-1-(2-hydroxyethyl)pyrazol / 1-Methoxy-2-amino-4-(2-hydroxyethylamino)benzol; 4,5-Diamino-1-(2-hydroxyethyl)pyrazol / 2-Amino-3-hydroxypyridin; 4,5-Diamino-1-(2-hydroxyethyl)pyrazol / 1-Naphthol.

Um eine ausgewogene und subtile Nuancenausbildung zu erzielen, ist es erfindungsgemäß vorteilhaft, wenn weitere farbgebende Komponenten im erfindungsgemäßen Mittel enthalten sind.

In einer weiteren Ausführungsform können die erfindungsgemäßen Mittel mindestens einen direktziehenden Farbstoff enthalten. Dabei handelt sich um Farbstoffe, die direkt auf das Haar aufziehen und keinen oxidativen Prozess zur Ausbildung der Farbe benötigen. Direktziehende Farbstoffe sind üblicherweise Nitrophenylendiamine, Nitroaminophenole, Azofarbstoffe, Anthrachinone oder Indophenole.

Die direktziehenden Farbstoffe werden jeweils bevorzugt in einer Menge von 0,001 bis 20 Gew.-%, insbesondere von 0,05 bis 5 Gew.-%, jeweils bezogen auf die gesamte Anwendungszubereitung, eingesetzt. Die Gesamtmenge an direktziehenden Farbstoffen beträgt vorzugsweise höchstens 3 Gew.-%.

Direktziehende Farbstoffe können in anionische, kationische und nichtionische direktziehende Farbstoffe unterteilt werden. Bevorzugte anionische direktziehende Farbstoffe sind die unter den internationalen Bezeichnungen bzw. Handelsnamen Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1 und Acid Black 52 bekannten Verbindungen. Bevorzugte kationische direktziehende Farbstoffe sind dabei

(a) kationische Triphenylmethanfarbstoffe, wie beispielsweise Basic Blue 7, Basic Blue 26, Basic Violet 2 und Basic Violet 14,

(b) aromatischen Systeme, die mit einer quaternären Stickstoffgruppe substituiert sind, wie beispielsweise Basic Yellow 57, Basic Red 76, Basic Blue 99, Basic Brown 16 und Basic Brown 17, sowie

(c) direktziehende Farbstoffe, die einen Heterocyclus enthalten, der mindestens ein quaternäres Stickstoffatom aufweist, wie sie beispielsweise in der EP-A2-998 908, auf die an dieser Stelle explizit Bezug genommen wird, in den Ansprüchen 6 bis 11 genannt werden. Die Verbindungen, die unter den Bezeichnungen Basic Yellow 87, Basic Orange 31 und Basic Red 51 bekannt sind, sind ganz besonders bevorzugte kationische direktziehende Farbstoffe der Gruppe (c).

Die kationischen direktziehenden Farbstoffe, die unter dem Warenzeichen Arianor® vertrieben werden, sind erfindungsgemäß ebenfalls ganz besonders bevorzugte kationische direktziehende Farbstoffe.

Als nichtionische direktziehende Farbstoffe eignen sich insbesondere nichtionische Nitro- und Chinonfarbstoffe und neutrale Azofarbstoffe. Bevorzugte nichtionische direktziehende Farbstoffe sind die unter den internationalen Bezeichnungen bzw. Handelsnamen HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9 bekannten Verbindungen,

sowie 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)-aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)-amino-5-chlor-2-nitrobenzol, 4-Amino-3-nitrophenol, 1-(2'-Ureidoethyl)amino-4-nitrobenzol, 2-[(4-Amino-2-nitrophenyl)amino]benzoesäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol.

Es ist nicht erforderlich, dass die fakultativ enthaltenen direktziehenden Farbstoffe jeweils einheitliche Verbindungen darstellen. Vielmehr können, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Weiterhin können als direktziehende Farbstoffe auch in der Natur vorkommende Farbstoffe eingesetzt werden, wie sie beispielsweise in Henna rot, Henna neutral, Henna schwarz, Kamillenblüte, Sandelholz, schwarzem Tee, Walnuss, Faulbaumrinde, Salbei, Blauholz, Krappwurzel, Catechu und Alkannawurzel enthalten sind.

Als Farbstoffvorstufen naturanaloger Farbstoffe werden bevorzugt solche Indole und Indoline eingesetzt, die mindestens zwei Gruppen ausgewählt aus Hydroxy- und/oder oder Aminogruppen, bevorzugt als Substituent am Sechsring, aufweisen. Diese Gruppen können weitere Substituenten tragen, z. B. in Form einer Veretherung oder Veresterung der Hydroxygruppe oder eine Alkylierung der Aminogruppe. In einer weiteren Ausführungsform enthalten die Färbemittel mindestens ein Indol- und/oder Indolinderivat. Erfindungsgemäße Zusammensetzungen, die Vorstufen naturanaloger Farbstoffe enthalten, werden bevorzugt als luftoxidative Färbemittel verwendet. In dieser Ausführungsform werden die besagten Zusammensetzungen folglich nicht mit einem zusätzlichen Oxidationsmittel versetzt.

Die Farbstoffvorstufen naturanaloger Farbstoffe werden jeweils bevorzugt in einer Menge von 0,001 bis 5 Gew.-%, bezogen auf die gesamte Anwendungszubereitung, eingesetzt. Besonders gut als Vorstufen naturanaloger Haarfarbstoffe geeignet sind Derivate des 5,6-Dihydroxyindolins, insbesondere 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin sowie 5,6-Dihydroxyindolin-2-carbonsäure, insbesondere 5,6-Dihydroxyindolin. Als Vorstufen naturanaloger Haarfarbstoffe hervorragend geeignet sind weiterhin Derivate des 5,6-Dihydroxyindols, insbesondere 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, insbesondere 5,6-Dihydroxyindol.

Im Falle der oxidativen Färbungen kann die Entwicklung der Farbe grundsätzlich mit Luftsauerstoff erfolgen. Bevorzugt wird jedoch ein chemisches Oxidationsmittel eingesetzt, besonders dann, wenn neben der Färbung ein Aufhelleffekt an menschlichem Haar gewünscht ist. Dieser Aufhelleffekt kann unabhängig von der Färbemethode gewünscht sein. Als Oxidationsmittel kommen Persulfate, Peroxodisulfate, Chlorite, Hypochlorite und insbesondere Wasserstoffperoxid oder und/oder eines seiner festen Anlagerungsprodukte an organische oder anorganische Verbindungen in Frage.

Um eine vorzeitige, unerwünschte Reaktion der Oxidationsfarbstoffvorprodukte durch das Oxidationsmittel zu verhindern, werden Oxidationsfarbstoffvorprodukte und Oxidationsmittel selbst zweckmäßigerweise getrennt voneinander konfektioniert und erst unmittelbar vor der Anwendung in Kontakt gebracht.

In einer weiteren Ausführungsform der vorliegenden Erfindung sind daher Mittel bevorzugt, welche unmittelbar vor der Anwendung durch Vermischen mindestens zweier Zubereitungen hergestellt werden, wobei die mindestens zwei Zubereitungen in mindestens zwei getrennt konfektionierten Containern bereitgestellt werden und wobei ein Container ein Färbemittel (A), welches in einem kosmetischen Träger mindestens ein Oxidationsfarbstoffvorprodukt enthält, und ein weiterer Container eine Oxidationsmittelzubereitung (B), enthaltend mindestens ein Oxidationsmittel, enthält, dadurch gekennzeichnet sind, dass mindestens eine der beiden Zubereitungen (A) oder (B) ein amphoteres Tensid gemäß Formel (I) enthält.

Bevorzugt enthält die Oxidationsmittelzubereitung (B) als Oxidationsmittel Wasserstoffperoxid und/oder eines seiner festen Anlagerungsprodukte an organische oder anorganische Verbindungen, wie Harnstoff, Melamin sowie Natriumborat.

Bevorzugt beträgt die Menge an Oxidationsmittel im anwendungsbereiten Mittel 0,5 bis 12 Gew.-%, bevorzugt 2 bis 10 Gew.-% insbesondere bevorzugt zu 3 bis 6 Gew.-% (berechnet als 100 %-iges $H_2O_2$), jeweils bezogen auf das anwendungsbereite Mittel.

Solche Oxidationsmittelzubereitungen sind vorzugsweise wässrige, fließfähige Oxidationsmittelzubereitungen. Dabei sind bevorzugte Zubereitungen dadurch gekennzeichnet, dass die fließfähige Oxidationsmittelzubereitung - bezogen auf ihr Gewicht - 40 bis 90 Gew.-%, vorzugsweise 50 bis 85 Gew.-%, besonders bevorzugt 55 bis 80 Gew.-%, weiter bevorzugt 60 bis 77,5 Gew.-% und insbesondere 65 bis 75 Gew.-% Wasser enthält.

Erfindungsgemäß kann aber das Oxidationsfärbemittel auch zusammen mit einem Katalysator auf das Haar aufgebracht werden, der die Oxidation der Farbstoffvorprodukte, z.B. durch Luftsauerstoff, aktiviert. Solche Katalysatoren sind z. B. bestimmte Enzyme, Iodide, Chinone oder Metallionen.

Weiterhin hat es sich als vorteilhaft erweisen, wenn die Oxidationsmittelzubereitungen mindestens einen Stabilisator oder Komplexbildner enthalten. Besonders bevorzugte Stabilisatoren sind Phenacetin, Alkalibenzoate (Natriumbenzoat) und Salicylsäure.

Erfindungsgemäß bevorzugt ist auch der Einsatz von sogenannten Komplexbildnern. Komplexbilder sind Stoffe, die Metallionen komplexieren können. Bevorzugte Komplexbildner sind sogenannte Chelatkomplexbildner, also Stoffe, die mit Metallionen cyclische Verbindungen bilden, wobei ein einzelner Ligand mehr als eine Koordinationsstelle an einem Zentralatom besetzt, d. h. mindestens "zweizähnig" ist. Die Zahl der gebundenen Liganden hängt von der Koordinationszahl des zentralen Ions ab. Erfindungsgemäß bevorzugte Komplexbildner sind stickstoffhaltigen Polycarbonsäuren, insbesondere EDTA, und Phosphonate, vorzugsweise Hydroxyalkan- bzw. Aminoalkanphosphonate und insbesondere 1-Hydroxyethan-1,1-diphosphonat (HEDP) bzw. dessen Di- oder Tetranatriumsalz und/oder Ethylendiamintetramethylenphosphonat (EDTMP) bzw. dessen Hexanatriumsalz und/oder Diethylentriaminpentamethylenphosphonat (DTPMP) bzw. dessen Hepta- oder Octanatriumsalz.

Die Färbezubereitung und gegebenenfalls Oxidationsmittelzubereitung enthalten weitere Hilfs- und Zusatzstoffe. So hat es sich erfindungsgemäß als bevorzugt erwiesen, wenn die Färbezubereitung und/oder die Oxidationsmittelzubereitung mindestens ein Verdickungsmittel enthält. Bezüglich dieser Verdickungsmittel bestehen keine prinzipiellen Einschränkungen. Es können sowohl organische als auch rein anorganische Verdickungsmittel zum Einsatz kommen.

Zur weiteren Steigerung der Leistung der Oxidationsmittelzubereitung können der erfindungsgemäßen Zusammensetzung zusätzlich mindestens eine gegebenenfalls hydratisierte $SiO_2$-Verbindung zugesetzt. Es kann erfindungsgemäß bevorzugt sein, die gegebenenfalls hydratisierten $SiO_2$-Verbindungen in Mengen von 0,05 Gew.-% bis 15 Gew.-%, besonders bevorzugt in Mengen von 0,15 Gew.-% bis 10 Gew.-% und ganz besonders bevorzugt in Mengen von 0,2 Gew.-% bis 5 Gew.-%, jeweils bezogen auf die erfindungsgemäße wasserfreie Zusammensetzung, einzusetzen. Die Mengenangaben geben dabei jeweils den Gehalt der $SiO_2$-Verbindungen (ohne deren Wasseranteil) in den Mitteln wieder. Erfindungsgemäß bevorzugt werden die $SiO_2$-Verbindunge in Form von Kieselgelen (Silicagel) oder besonders bevorzugt als Wasserglas eingesetzt. Diese $SiO_2$-Verbindungen können teilweise in wässriger Lösung vorliegen. Erfindungsgemäß besonders bevorzugte Wassergläser werden unter anderem von der Firma Henkel unter den Bezeichnungen Ferrosil® 119, Natronwasserglas 40/42, Portil® A, Portil® AW und Portil® W und von der Firma Akzo unter der Bezeichnung Britesil® C20 vertrieben.

Vorzugsweise sind die Zubereitung (A) und/oder gegebenenfalls die Oxidationsmittelzubereitung (B) als fließfähigen Zubereitungen konfektioniert.

Vorzugsweise wird den fließfähigen Zubereitungen (A) und/oder (B) zusätzlich ein Emulgator bzw. ein weiteres Tensid zugesetzt, wobei oberflächenaktive Substanzen je nach Anwendungsgebiet als Tenside oder als Emulgatoren bezeichnet werden und aus anionischen, kationischen, zwitterionischen, amphoteren und nichtionischen Tensiden und Emulgatoren ausgewählt sind. Diese Stoffe werden nachfolgend ausführlich beschrieben.

Ein erfindungsgemäß besonders bevorzugtes Mittel ist dadurch gekennzeichnet, dass das Mittel zusätzlich mindestens ein anionisches Tensid enthält. Als anionische Tenside eignen sich in erfindungsgemäßen Zubereitungen alle für die Verwendung am menschlichen Körper geeigneten anionischen oberflächenaktiven Stoffe. Bevorzugte anionische Tenside sind Alkylsulfate, Alkylethersulfate und Ethercarbonsäuren mit 10 bis 20 C-Atomen in der Alkylgruppe und bis zu 16 Glykolethergruppen im Molekül. Die anionischen Tenside werden in Mengen von 0,1 bis 45 Gew.%, bevorzugt 1 bis 30 Gew.% und ganz besonders bevorzugt von 1 bis 15 Gew.%, bezogen auf die Gesamtmenge des anwendungsbereiten Mittels, eingesetzt.

Als zwitterionische Tenside werden solche oberflächenaktiven Verbindungen bezeichnet, die im Molekül mindestens eine quartäre Ammoniumgruppe und mindestens eine Carboxylat-, Sulfonat- oder Sulfat-Gruppe tragen. Besonders geeignete zwitterionische Tenside sind die sogenannten Betaine wie die N-Alkyl-N,N-dimethylammonium-glycinate, N-Acyl-aminopropyl-N,N-dimethylammoniumglycinate, und 2-Alkyl-3-carboxymethyl-3-hydroxyethyl-imidazoline mit jeweils 8 bis 18 C-Atomen in der Alkyl- oder Acylgruppe sowie Kokosacylaminoethylhydroxyethylcarboxymethylglycinat. Ein bevorzugtes zwitterionisches Tensid ist Cocamidopropyl Betaine.

Beispiele für geeignete amphotere Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Besonders bevorzugte amphotere Tenside sind das N-Kokosalkylaminopropionat, das Kokosacylaminoethylaminopropionat und das $C_{12}$-$C_{18}$-Acylsarcosin.

Weiterhin hat es sich als vorteilhaft erwiesen, wenn die erfindungsgemäßen Färbemittel weitere, nichtionogene grenzflächenaktive Stoffe, enthalten. Nichtionische Tenside enthalten als hydrophile Gruppe z. B. eine Polyolgruppe, eine Polyalkylenglykolethergruppe oder eine Kombination aus Polyol- und Polyglykolethergruppe. Als nichtionische Tenside eignen sich insbesondere $C_8$-$C_{22}$-Alkylmono- und oligoglycoside und deren ethoxylierte Analoga. Als weitere bevorzugte nichtionische Tenside haben sich die Alkylenoxid-Anlagerungsprodukte an gesättigte lineare Fettalkohole und Fettsäuren mit jeweils 2 bis 30 Mol Ethylenoxid pro Mol Fettalkohol bzw. Fettsäure erwiesen. Zubereitungen mit hervorragenden Eigenschaften werden ebenfalls erhalten, wenn sie als nichtionische Tenside Fettsäureester von ethoxyliertem Glycerin enthalten.

Die zusätzlichen nichtionischen, zwitterionischen oder amphoteren Tenside werden in Mengen von 0,1 bis 45 Gew.%, bevorzugt 1 bis 30 Gew.% und ganz besonders bevorzugt von 1 bis 15 Gew.%, bezogen auf die Gesamtmenge des anwendungsbereiten Mittels, eingesetzt.

Erfindungsgemäß bevorzugt sind ebenfalls kationische Tenside vom Typ der quartären Ammoniumverbindungen, der Esterquats und der Amidoamine. Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf. Weitere erfindungsgemäß verwendbare kationische Tenside stellen die quaternisierten Proteinhydrolysate dar. Eine erfindungsgemäß besonders geeignete Verbindung der Alkylamidoamine stellt das unter der Bezeichnung Tegoamid® S 18 im Handel erhältliche Stearamidopropyl-dimethylamin dar. Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats werden beispielsweise unter den Warenzeichen Stepantex®, Dehyquart® und Armocare® vertrieben.

Die kationischen Tenside sind in den erfindungsgemäß verwendeten Mitteln bevorzugt in Mengen von 0,05 bis 10 Gew.-%, bezogen auf das gesamte Mittel, enthalten. Mengen von 0,1 bis 5 Gew.-% sind besonders bevorzugt.

In einer bevorzugten Ausführungsform können nicht-ionische, zwitterionische und/oder amphotere Tenside sowie deren Mischungen bevorzugt sein.

In einer weiteren bevorzugten Ausführungsform kann die Wirkung des erfindungsgemäßen Wirkstoffes durch Emulgatoren gesteigert werden. Solche Emulgatoren sind beispielsweise

- Anlagerungsprodukte von 4 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- $C_{12}$-$C_{22}$-Fettsäuremono- und -diester von Anlagerungsprodukten von 1 bis 30 Mol Ethylenoxid an Polyole mit 3 bis 6 Kohlenstoffatomen, insbesondere an Glycerin,
- Ethylenoxid- und Polyglycerin-Anlagerungsprodukte an Methylglucosid-Fettsäureester, Fettsäurealkanolamide und Fettsäureglucamide,
- Gemische aus Alkyl-(oligo)-glucosiden und Fettalkoholen zum Beispiel das im Handel erhältliche Produkt Montanov® 68,
- Anlagerungsprodukte von 5 bis 60 Mol Ethylenoxid an Rizinusöl und gehärtetes Rizinusöl,
- Partialester von Polyolen mit 3 bis 6 Kohlenstoffatomen mit gesättigten Fettsäuren mit 8 bis 22 C-Atomen,
- Phospholipide, vor allem Glucose-Phospolipide, die z. B. als Lecithine bzw. Phosphatidylcholine aus z.B. Eidotter oder Pflanzensamen (z.B. Sojabohnen) gewonnen werden,
- Fettsäureester von Zuckern und Zuckeralkoholen, wie Sorbit
- Polyglycerine und Polyglycerinderivate wie beispielsweise Polyglycerinpoly-12-hydroxystearat (Handelsprodukt Dehymuls® PGPH)
- Lineare und verzweigte Fettsäuren mit 8 bis 30 C-Atomen und deren Na-, K-, Ammonium-, Ca-, Mg- und Zn-Salze.

Die erfindungsgemäßen Mittel enthalten die Emulgatoren bevorzugt in Mengen von 0,1 bis 25 Gew.-%, insbesondere 0,5 bis 15 Gew.-%, bezogen auf die Gesamtmenge des anwendungsbereiten Mittel. Nichtionogene Emulgatoren bzw. Tenside mit einem HLB-Wert von 10-15 können erfindungsgemäß besonders bevorzugt sein. Unter den genannten Emulgatoren-Typen können die Emulgatoren, welche kein Ethylenoxid und/oder Propylenoxid im Molekül enthalten ganz besonders bevorzugt sein.

Ferner können die erfindungsgemäßen Mittel weitere Wirk-, Hilfs- und Zusatzstoffe enthalten, beispielsweise nichtionische Polymere (wie Vinylpyrrolidinon/Vinylacrylat-Copolymere, Polyvinylpyrrolidinon, Vinylpyrrolidinon/Vinylacetat-Copolymere, Polyethylenglykole und Polysiloxane); Silikone (wie Dimethicone, Cyclomethicone, Polyarylsiloxane und/oder Polyalkylarylsiloxane, Amodimethicone, Dimethiconcopolyole); kationische Polymere (wie quaternisierte Celluloseether, Polysiloxane mit quaternären Gruppen, Dimethyldiallylammoniumchlorid-Polymere, Acrylamid-Dimethyldiallyl-ammoniumchlorid-Copolymere, mit Diethylsulfat quaternierte Dimethylamino-ethylmethacrylat-Vinylpyrrolidinon-Copolymere, Vinylpyrrolidinon-Imidazoliniummethochlorid-Copolymere und quaternierter Polyvinylalkohol); zwitterionische und amphotere Polymere (wie Acrylamidopropyl-trimethylammoniumchlorid/Acrylat-Copolymere und Octylacrylamid/Methyl-methacrylat/tert-Butylaminoethylmethacrylat/2-Hydroxypropylmethacrylat-Copolymere, Diallyldimethylammoniumchlorid/Acrylat-Copolymere, t-Butylaminoethylmethacrylat/N-(1,1,3,3-Tetramethylbutyl)acrylamid/Acrylat(/Methacrylat)-Copolymere); anionische Polymere (wie Polyacrylsäuren, vernetzte Polyacrylsäuren, Vinylacetat/Crotonsäure-Copolymere, Vinylpyrrolidinon/Vinylacrylat-Copolymere, Vinylacetat/Butylmaleat/Isobornylacrylat-Copolymere, Methylvinylether/Malein-säureanhydrid-Copolymere und Acrylsäure/Ethylacrylat/N-t-Butyl-acrylamid-Terpolymere);

weitere Verdickungsmittel (wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z. B. Polyvinylalkohol); Strukturanten (wie Glucose, Maleinsäure und Milchsäure); haarkonditionierende Verbindungen (wie Phospholipide, beispielsweise Sojalecithin, Ei-Lecitin und Kephaline); Parfümöle und Cyclodextrine; faserstrukturverbessernde Wirkstoffe (wie Glucose, Galactose, Fructose, Fruchtzucker und Lactose); Antischuppenwirkstoffe (wie Piroctone Olamine, Zink Omadine und Climbazol); Aminosäuren und Oligopeptide, (insbesondere Arginin und/oder Serin); Proteinhydrolysate (wie beispielsweise Elastin-, Kollagen-, Keratin-, Seiden- und Milcheiweiß-Proteinhydrolysate, oder Mandel-, Reis-, Erbsen-, Kartoffel- und Weizenproteinhydrolysate, sowie in Form ihrer Fettsäure-Kondensationsprodukte oder gegebenenfalls anionisch oder kationisch modifizierten Derivate); pflanzliche Öle (wie Macadamianussöl, Kukuinussöl, Palmöl, Amaranthsamenöl, Pfirsichkernöl, Avocadoöl, Olivenöl, Kokosöl, Rapsöl, Sesamöl, Jojobaöl, Sojaöl, Erdnussöl, Nachtkerzenöl und Teebaumöl); Lichtschutzmittel (wie derivatisierte Benzophenone, Zimtsäure-Derivate und Triazine); Wirkstoffe (wie Panthenol, Pantothensäure, Pantolacton, Allantoin, Pyrrolidinoncarbonsäuren und deren Salze sowie Bisabolol); Pflanzenextrakte (wie die Extrakte aus Aloe Vera, Angelica, Anis, Aprikose, Benzoe, Bergamotte, Birke, Brennnessel, Calmus, Cassis, Costus, Eibisch, Eichenrinde, Elemi, Estragon, Fichtennadel, Galbanum, Geranium, Ginseng, Grapefruit, Guajakholz , grünem Tee, Hamamelis, Hauhechel, Hopfen, Huflattich, Ingwerwurzel, Iris, Jasmin, Kamille, Kardamon, Klee, Klettenwurzel, Kiefer, Kiwi, Kokosnuss, Koriander, Kümmel, Latschen, Lavendel, Lemongras, Lilie, Limone, Lindenblüten, Litchi, Macis, Malve, Mandel, Mango, Melisse, Melone, Meristem, Myrrhe, Neroli, Olibanum, Opoponax, Orange, Patchouli, Petitgrain, Pinie, Quendel, Rooibos, Rosen, Rosmarin, Rosskastanie, Sandelholz, Salbei, Schachtelhalm, Schafgarbe, Sellerie, Tanne, Thymian, Wacholder, Weinblättern, Weißdorn, Weizen, Wiesenschaumkraut, Ylang-Ylang, Zeder und Zitrone); Konsistenzgeber (wie Zuckerester, Polyolester oder Polyolalkylether); Fette und Wachse (wie Bienenwachs, Montanwachs und Paraffine); Quell- und Penetrationsstoffe (wie Glycerin, Propylenglykolmonoethylether, Carbonate, Hydrogencarbonate, Guanidine, Harnstoffe sowie primäre, sekundäre und tertiäre Phosphate); Trübungsmittel (wie Latex, Styrol/PVP- und Styrol/Acrylamid-Copolymere); Perlglanzmittel (wie Ethylenglykolmono- und -distearat sowie PEG-3-distearat); Treibmittel (wie Propan-Butan-Gemische, $N_2O$, Dimethylether, $CO_2$ und Luft) sowie Antioxidantien.

Die Auswahl dieser weiteren Stoffe wird der Fachmann gemäß der gewünschten Eigenschaften der Mittel treffen. Bezüglich weiterer fakultativer Komponenten sowie der eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen. Die zusätzlichen Wirk- und Hilfsstoffe werden in den erfindungsgemäßen Mitteln bevorzugt in Mengen von jeweils 0,0001 bis 10 Gew.-%, insbesondere von 0,0005 bis 5 Gew.-%, bezogen auf das Gesamtgewicht der Anwendungsmischung, eingesetzt.

Wird gleichzeitig zu der Färbung der keratinischen Faser eine starke Aufhellung gewünscht, so ist daher es erfindungsgemäß bevorzugt, wenn zusätzlich eine Blondierzubereitung (C), enthaltend mindestens einen Bleichaktivator, der Mischung aus Oxidationsmittelzubereitung (B) und der Färbezubereitung (A), enthaltend in einem kosmetischen Träger mindestens eine farbgebende Komponente, beigemischt wird, wobei mindestens eine Zubereitungen (A), (B) oder (C) ein amphoteres Tensid gemäß Formel (I) enthält. Bevorzugt enthält die Färbezubereitung (A) das Tensid gemäß Formel (I). Es kann dabei unerheblich sein, ob zunächst eine Mischung aus (A) und (B) hergestellt wird, und anschließend die Blondierzubereitung (C) zugemischt wird, oder ob eine davon verschiedene Reihenfolge der Vermischung der einzelnen Komponenten genutzt wird. Es ist bevorzugt, die einzelnen Zubereitungen in möglichst naher zeitlicher Abfolge zu vermischen und das anwendungsbereite Mittel vorzugsweise zeitnah auf die keratinischen Fasern zu applizieren.

Eine weitere Ausführungsform der vorliegenden Anmeldung ist daher ein Mittel zum Bleichen und Färben keratinischer Fasern, dadurch gekennzeichnet, dass es durch Vermischen von mindestens einer Oxidationsmittelzubereitung (B), enthaltend mindestens ein Oxidationsmittel, ausgewählt aus Wasserstoffperoxid dessen Anlagerungsverbindungen an feste Träger, mindestens einer Blondierzubereitung (C), enthaltend mindestens einen Bleichkraftverstärker, und mindestens einer Färbezubereitung (A), enthaltend in einem kosmetischen Träger mindestens ein Oxidationsfarbstoffvorprodukt sowie mindestens ein amphoteres Tensid gemäß Formel (I), vor der Anwendung hergestellt wird.

In einer weiteren Ausführungsform ist es bevorzugt, wenn das erfindungsgemäße Färbemittel zusätzlich als Blondierzubereitung (C) mindestens eine anorganische Peroxoverbindung enthält. Vorzugsweise ist die anorganische Peroxoverbindung ausgewählt aus Ammoniumperoxodisulfat, Alkalimetallperoxodisulfaten, Ammoniumperoxomonosulfat, Alkalimetallhydrogenperoxomonosulfaten, Alkalimetallperoxodiphosphaten und Erdalkalimetallperoxiden. Besonders bevorzugte anorganische Peroxoverbindung als Bleichkraftverstärker sind Ammoniumperoxodisulfat, Kaliumperoxodisulfat, Natriumperoxodisulfat, Kaliumhydrogenperoxomonosulfat, Kaliumperoxodiphosphat, Magnesiumperoxid und Bariumperoxid, insbesondere Ammoniumperoxodisulfat, Kaliumperoxodisulfat und Natriumperoxodisulfat. Die anorganischen Peroxo-Verbindungen sind vorzugsweise in einer Menge von 0,1 bis 25 Gew.-%, insbesondere in einer Menge von 0,5 bis 15 Gew.-%, bezogen auf das Gesamtgewicht des anwendungsbereiten Mittels, enthalten. Der Einsatz von Persulfatsalzen bzw. Peroxodisulfatsalzen erfolgt in der Regel in Form eines gegebenenfalls entstaubten Pulvers oder eines in Form gepressten Formkörpers.

Es kann jedoch erfindungsgemäß vorteilhaft sein, wenn die Mittel frei von anorganischen Peroxoverbindungen sind. Die erfindungsgemäßen Mittel können daher jedoch anstelle und/oder zusätzlich zu den festen Peroxoverbindungen einen weiteren Bleichkraftverstärker enthalten. Als zusätzliche Bleichkraftverstärker können im Rahmen dieser Erfindung Verbindungen, die unter Perhydrolysebedingungen aliphatische Peroxocarbonsäuren und/oder substituierte Perbenzoesäure ergeben, Kohlensäurederivate, Alkylcarbonate und -carbamate sowie Silylcarbonate und - carbamate eingesetzt werden.

Obwohl prinzipiell keine Einschränkungen hinsichtlich der Formulierung der Blondierzubereitung (C) bestehen, hat es sich erfindungsgemäß als bevorzugt erwiesen, wenn die Zubereitung (C) wasserfrei formuliert ist. Wasserfrei im Sinne der vorliegenden Erfindung bedeutet einen Wassergehalt bezogen auf die Zubereitung (C) von weniger als 5 Gew.-%, insbesondere von weniger als 2 Gew.-%. Blondierzubereitungen, die weniger als 0,1 Gew.-% Wasser, enthalten können erfindungsgemäß ganz besonders bevorzugt sein. Die Zubereitung (C) ist vorzugsweise als Pulver oder als wasserfreie Paste formuliert.

In einer weiteren, bevorzugten Ausführungsform kann das Mittel in der Zubereitung (C) als Bleichkraftverstärker mindestens ein kationisches Pyridinium-Derivat enthalten. In einer besonders bevorzugten Ausführungsform enthalten die erfindungsgemäßen Mittel als kationisches Pyridinium-Derivat gemäß Formel (II) eine Verbindung, ausgewählt aus 2-Acetyl-1-methylpyridinium-p-toluolsulfonat, 4-Acetyl-1-methylpyridinium-p-toluolsulfonat und N-Methyl-3,4-dihydroisochinolinium-p-toluolsulfonat.

Eine erfindungsgemäß bevorzugte Ausführungsform der vorliegenden Erfindung besteht darin, dass das anwendungsbereite Mittel einen pH- zwischen 6 und 11, insbesondere zwischen 7 und 10,5, insbesondere bevorzugt zwischen 8 und 10, besitzt. Bei den pH-Werten im Sinne der vorliegenden Erfindung handelt es sich um pH-Werte, die bei einer Temperatur von 22 °C gemessen wurden.

Üblicherweise wird der pH-Wert mit pH-Stellmitteln eingestellt. Zur Einstellung des pH-Werts sind dem Fachmann in der Kosmetik gängige Acidifizierungs- und Alkalisierungsmittel geläufig. Die zur Einstellung des pH-Wertes verwendbaren Alkalisierungsmittel werden typischerweise gewählt aus anorganischen Salzen, insbesondere der Alkali- und Erdalkalimetalle, organischen Alkalisierungsmitteln, insbesondere Aminen, basische Aminosäuren und Alkanolaminen, und Ammoniak. Erfindungsgemäß bevorzugte Acidifizierungsmittel sind Genuss-Säuren, wie beispielsweise Zitronensäure, Essigsäure, Äpfelsäure oder Weinsäure, sowie verdünnte Mineralsäuren. Erfindungsgemäß besonders bevorzugte Alkanolamine werden ausgewählt aus der Gruppe 2-Aminoethan-1-ol (Monoethanolamin), 2-Amino-2-methylpropan-1-ol, 2-Amino-2-methyl-propan-1,3-diol und Triethanolamin. Das erfindungsgemäße, anorganische Alkalisierungsmittel wird bevorzugt ausgewählt aus Natriumhydroxid, Kaliumhydroxid, Calciumhydroxid, Bariumhydroxid, Natriumphosphat, Kaliumphosphat, Natriumsilicat, Kaliumsilicat, Natriumcarbonat und Kaliumcarbonat. Die als erfindungsgemäßes Alkalisierungsmittel einsetzbaren basischen Aminosäuren werden bevorzugt ausgewählt aus der Gruppe, die gebildet wird aus L-Arginin, D-Arginin, D/L-Arginin, L-Lysin, D-Lysin, D/L-Lysin. Schließlich ist ein weiteres bevorzugtes Alkalisierungsmittel Ammoniak. Bevorzugt werden die Alkalisierungsmittel in einer Menge von 0,05 bis 10 Gew.-%, insbesondere von 0,5 bis 5 Gew.-%, jeweils bezogen auf das Gesamtgewicht des anwendungsbereiten Mittels, enthalten.

Eine weitere bevorzugte Ausführungsform der vorliegenden Erfindung ist insbesondere dadurch gekennzeichnet, dass das Mittel ammoniakarm, bevorzugt ammoniakfrei ist. Dadurch kann eine Reduzierung von Gerüchen, die dem Verbraucher unangenehm auffallen, und zusätzlich eine Verringerung des Reizpotentials, welches von ammoniakhaltigen Mitteln ausgeht, erzielt werden. Die Begriffe "ammoniakarm" bzw. "ammoniakfrei" im Sinne der Erfindung beziehen sich dabei auf die dem Mittel zugesetzte Ammoniakmenge, wobei der Ammoniakzusatz sowohl als wässrige, alkoholische, wässrig-alkoholische oder anderweitige Lösung als auch durch Ammoniakgaseinleitung oder durch Zusatz von verflüssigtem Ammoniak erfolgen kann. Der Zusatz von Ammoniak kann aber auch durch Verwendung von Ammonium-Salzen erfolgen, wobei das AmmoniumKation je nach pH-Wert der Zubereitung im Gleichgewicht mit seiner korrespondierenden Base, dem Ammoniak selbst, steht. Die Begriffe "ammoniakfrei" bzw. "ammoniakarm" im Sinne der Erfindung beziehen sich daher auch auf Mittel, die Ammonium-Salze in den entsprechenden Mengen enthalten. Bevorzugte, ammoniakarme Mittel enthalten weniger als 2 Gew.-%, insbesondere weniger als 0,5 Gew.-% und ganz besonders bevorzugt weniger als 0,1 Gew.-% zugesetzten Ammoniak, jeweils bezogen auf das Gesamtgewicht der Anwendungszubereitung. Ammoniakfrei im Sinne der Erfindung sind solche Mittel, denen kein Ammoniak durch eine der oben beschriebenen Methoden zugesetzt wurde. Solche Mittel sind ganz besonders bevorzugt.

Wie bereits erwähnt, können die erfindungsgemäßen Mittel auch direkt vor der Anwendung aus zwei oder mehreren getrennt verpackten Zubereitungen hergestellt werden. Dies bietet sich insbesondere zur Trennung inkompatibler Inhaltsstoffe an, um eine vorzeitige Reaktion zu vermeiden. Eine Auftrennung in Mehrkomponentensysteme bietet sich insbesondere dort an, wo Inkompatibilitäten der Inhaltsstoffe zu erwarten oder zu befürchten sind. Das anwendungsbereite Mittel wird bei solchen Systemen vom Verbraucher direkt vor der Anwendung durch Vermischen der Komponenten hergestellt. Ein Färbe- und/oder Aufhellungsmittel, bei dem die Oxidationsfarbstoffvorprodukte zunächst getrennt von der Oxidationsmittelzubereitung, enthaltend bevorzugt Wasserstoffperoxid, vorliegen, ist dabei bevorzugt.

Eine bevorzugte Darreichungsform des erfindungsgemäßen Mittels ist eine Verpackungseinheit (Kit-of-Parts),

welche in getrennt voneinander konfektionierten Containern

- in einem Container A mindestens eine Zubereitung (A) enthält, enthaltend in einem kosmetischen Träger mindestens eine farbgebende Komponente und mindestens ein amphoteres Tensid gemäß Formel (I), und
- in einem Container B mindestens eine Oxidationsmittelzubereitung (B), enthaltend in einem kosmetischen Träger mindestens ein Oxidationsmittel, enthält.

Eine besonders bevorzugte Darreichungsform des erfindungsgemäßen Mittels ist eine Verpackungseinheit (Kit-of-Parts), welche in getrennt voneinander konfektionierten Containern

- in einem Container A mindestens eine Zubereitung (A) enthält, enthaltend in einem kosmetischen Träger als farbgebende Komponente mindestens ein Oxidationsfarbstoffvorprodukt und mindestens ein amphoteres Tensid gemäß Formel (I), und
- in einem Container B mindestens eine Oxidationsmittelzubereitung (B), enthaltend in einem kosmetischen Träger mindestens ein Oxidationsmittel,

enthält.

Wird ein besonders starker Aufhelleffekt gewünscht, so ist eine bevorzugte weitere Darreichungsform des erfindungsgemäßen Mittels eine Verpackungseinheit (Kit-of-Parts), welche in getrennt voneinander konfektionierten Containern

- in einem Container A mindestens eine Zubereitung (A), enthaltend in einem kosmetischen Träger mindestens ein Oxidationsfarbstoffvorprodukt und mindestens ein amphoteres Tensid gemäß Formel (I),
- in einem Container B mindestens eine Oxidationsmittelzubereitung (B), enthaltend mindestens ein Oxidationsmittel, und
- in einem Container C mindestens eine Blondierzubereitung (C), enthaltend mindestens einen Bleichkraftverstärker, enthält.

Es hat sich gezeigt, dass das amphotere Tensid gemäß Formel (I) auch sehr gute Effekte hinsichtlich einer Egalisierung in sogenannten "leave-on"-Vorbehandlungsmitteln vor der eigentlichen Färbung erzielt.

Dazu wird eine Zubereitung (VM), enthaltend das amphotere Tensid gemäß Formel (I) in einem kosmetischen Träger auf die zu färbenden keratinischen Fasern aufgetragen. Nach einer Einwirkzeit von 1 bis 20 min, bevorzugt von 3 bis 10 min, wird dann ohne vorheriges Auswaschen der Fasern eine Färbezubereitung, enthaltend mindestens eine farbgebende Komponente, auf die Fasern aufgetragen. Bei der Färbezubereitung kann es sich um ein Mittel handeln, welches zuvor aus einer Färbezubereitung (A) und einer Oxidationsmittelzubereitung (B) sowie gegebenenfalls einer Blondierzubereitung (C) hergestellt wurde. Dabei kann es für die Erzielung besonders positiver Färbeergebnisse wiederum bevorzugt sein, wenn die Färbezubereitung neben der farbgebenden Komponente zusätzlich ein amphoteres Tensid gemäß Formel (I) enthält.

Die Vorbehandlungszubereitung (VM) enthält das erfindungsgemäße amphotere Tensid bevorzugt in einem kosmetischen Träger. Das erfindungsgemäße amphotere Tensid ist bevorzugt in einer Menge von 0,1 bis 10 Gew.-%, bezogen auf das Gesamtgewicht des Vorbehandlungsmittels, enthalten.

In einer weiteren Ausführungsform der vorliegenden Erfindung hat es sich als vorteilhaft erwiesen, wenn das Vorbehandlungsmittel (VM) zur Viskositätseinstellung ein verdickendes Polymer enthält. Hierzu können nichtionische, kationische, zwitterionische, amphotere oder anionische Polymere eingesetzt werden. Bevorzugte verdickungsmittel können dabei natürlichen Ursprungs sein, wie beispielsweise Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z. B. Polyvinylalkohol. Besonders bevorzugt ist dabei Xanthan.

Die verdickenden Polymere sind im Vorbehandlungsmittel (VM) bevorzugt in einer Menge von 0,1 bis 10 Gew.-%, bezogen auf das Gesamtgewicht des Vorbehandlungsmittels, enthalten.

Eine weitere bevorzugte Darreichungsform des erfindungsgemäßen Mittels ist daher eine Verpackungseinheit (Kit-of-Parts), welche in getrennt voneinander konfektionierten Containern

- in einem Container V mindestens ein Vorbehandlungsmittel (VM), enthaltend in einem kosmetischen Träger mindestens ein amphoteres Tensid gemäß Formel (I),
- in einem Container A mindestens eine Zubereitung (A) enthält, enthaltend in einem kosmetischen Träger mindestens eine farbgebende Komponente, und

- in einem Container B mindestens eine Oxidationsmittelzubereitung (B), enthaltend in einem kosmetischen Träger mindestens ein Oxidationsmittel, enthält.

Ganz besonders bevorzugt ist dabei eine Verpackungseinheit, welche in getrennt voneinander konfektionierten Containern

- in einem Container V mindestens ein Vorbehandlungsmittel (VM), enthaltend in einem kosmetischen Träger mindestens ein amphoteres Tensid gemäß Formel (I),
- in einem Container A mindestens eine Zubereitung (A) enthält, enthaltend in einem kosmetischen Träger mindestens eine farbgebende Komponente und mindestens ein amphoteres Tensid gemäß Formel (I), und
- in einem Container B mindestens eine Oxidationsmittelzubereitung (B), enthaltend in einem kosmetischen Träger mindestens ein Oxidationsmittel, enthält.

Gegebenenfalls enthalten die Verpackungseinheiten mit Vorbehandlungsmittel (VM) ebenfalls eine Blondierzubereitung (C).

Bevorzugt enthält die Mehrkomponentenverpackungseinheit (Kit-of-Parts) zusätzlich eine Gebrauchsanleitung. Darüber hinaus kann es bevorzugt sein, wenn weiterhin eine Applikationshilfe, wie ein Kamm oder ein Pinsel, und/oder eine persönliche Schutzausrüstung, beispielsweise Einweg-Handschuhe, dem Kit beigefügt ist. Bezüglich weiterer bevorzugter Ausführungsformen der Mehrkomponentenverpackungseinheit (Kit-of-Parts) gilt mutatis mutandis das zu den erfindungsgemäßen Mitteln Gesagte.

Das eigentliche Haarfärbemittel wird zweckmäßigerweise unmittelbar vor der Anwendung durch Mischung der Zubereitungen (A) mit (B) sowie gegebenenfalls (C) hergestellt. Die Anwendungstemperaturen können in einem Bereich zwischen 15 und 40 °C liegen. Nach einer Einwirkungszeit von 5 bis 45 Minuten wird das Haarfärbemittel durch Ausspülen von dem zu färbenden Haar entfernt. Das Nachwaschen mit einem Shampoo entfällt, wenn ein stark tensidhaltiger Träger, z.B. ein Färbeshampoo, verwendet wurde.

Ein weiterer Gegenstand der vorliegenden Erfindung ist daher ein Verfahren zum Färben und gegebenenfalls Aufhellen menschlicher Haare, bei dem ein erfindungsgemäßes Mittel gemäß obiger Vorgaben auf das Haar aufgetragen wird, für eine Einwirkzeit von 5 bis 45 Minuten, bevorzugt von 8 bis 35 Minuten, auf dem Haar belassen wird, und aus dem Haar ausgespült oder mit einem Shampoo ausgewaschen wird.

Während der Einwirkzeit des Mittels auf der Faser kann es vorteilhaft sein, den Färbevorgang durch Wärmezufuhr zu unterstützen. Die Wärmezufuhr kann durch eine externe Wärmequelle, wie z.B. warme Luft eines Warmluftgebläses, als auch, insbesondere bei einer Haarfärbung am lebenden Probanden, durch die Körpertemperatur des Probanden erfolgen. Bei letzterer Möglichkeit wird üblicherweise die zu färbende Partie mit einer Haube abgedeckt. Insbesondere liegt die Temperatur während der Einwirkzeit zwischen 10 °C und 40 °C, insbesondere zwischen 20 °C und 38 °C. Die erfindungsgemäßen Färbemittel ergeben bereits bei physiologisch verträglichen Temperaturen von unter 45°C intensive Färbungen. Sie eignen sich deshalb besonders zum Färben von menschlichen Haaren.

In einer weiteren Ausführungsform des erfindungsgemäßen Verfahrens wird zunächst ein Vorbehandlungsmittel (VM) als "Leave-on"-Zubereitung auf das Haar aufgetragen. Die Anwendungstemperaturen können in einem Bereich zwischen 15 und 40 °C liegen. Nach einer Einwirkungszeit von 1 bis 20 Minuten, bevorzugt nach 3 bis 10 min wird auf das Haar ohne voriges Auswaschen oder Spülen ein Färbemittel zugegeben, welches gegebenenfalls unmittelbar zuvor durch Vermischen einer Färbezubereitung (A) mit einer Oxidationsmittelzubereitung (B) sowie gegebenenfalls einer Blondierzubereitung (C) hergestellt wurde. Gemäß vorliegender Erfindung enthält das Vorbehandlungsmittel (VM) und/oder das Färbemittel mindestens ein amphoteres Tensid gemäß Formel (I). Nach einer Einwirkzeit von 5 bis 45 Minuten, bevorzugt von 8 bis 35 Minuten, wird das Haar ausgespült oder mit einem Shampoo ausgewaschen wird. Das Nachwaschen mit einem Shampoo entfällt, wenn ein stark tensidhaltiger Träger, z.B. ein Färbeshampoo, verwendet wurde.

Ein weiterer Gegenstand der Erfindung ist die kosmetische Verwendung einer Verbindung gemäß Formel(I) und/oder eines ihrer physiologisch verträglichen Salze zur Färbung keratinischer Fasern, insbesondere menschlicher Haare.

Bevorzugt ist Verwendung eines Mittels zur Verbesserung der Grauabdeckung, insbesondere am Haaransatz, bei der oxidativen Farbveränderung menschlicher Haare. Weiterhin bevorzugt ist kosmetische Verwendung einer Verbindung gemäß Formel (I) und/oder eines ihrer physiologisch verträglichen Salze zur Verbesserung der Egalisierung von Färbungen auf keratinischen Fasern. Insbesondere bevorzugt ist kosmetische Verwendung einer Verbindung gemäß Formel (I) und/oder eines ihrer physiologisch verträglichen Salze zur Verbesserung der Waschbeständigkeit von Färbungen auf keratinischen Fasern.

Bezüglich weiterer bevorzugter Ausführungsformen der erfindungsgemäßen Verfahren und Verwendungen gilt mutatis mutandis das zu den erfindungsgemäßen Mitteln Gesagte.

Die nachfolgenden Beispiele sollen die Erfindung beispielhaft darstellen, ohne sie jedoch darauf zu beschränken

und im Schutzumfang einzuschränken.

**Beispiele**

**1. Zubereitungen**

a) Vorbehandlungsmittel (VM1)

| Rohstoff | VM1 [Gew.-%] |
|---|---|
| Produkt T4835 | 10,0 |
| Xanthan | 2,0 |
| 1,2-Propandiol | 4,0 |
| Wasser, vollentsalzt | ad 100 |

b) Färbecremes

| Rohstoff | V1 [Gew.-%] | E1 [Gew.-%] |
|---|---|---|
| Lanette D | 6,60 | 6,60 |
| Lorol C12-18 techn. | 2,40 | 2,40 |
| Eumulgin B 2 | 0,60 | 0,60 |
| Eumulgin B 1 | 0,60 | 0,60 |
| Lamesoft PO 65 | 2,00 | 2,00 |
| Akypo Soft 45HP | 10,0 | 10,0 |
| Texapon K 14 S Special 70 % | 2,80 | 2,80 |
| Produkt W 37194 | 3,75 | 3,75 |
| Produkt T4835 | -- | 5,00 |
| p-Toluylendiaminsulfat | 0,37 | 0,37 |
| Resorcin | 0,10 | 0,10 |
| 2-Methylresorcin | 0,90 | 0,90 |
| 1-(2-Hydroxyethylamino)-4-methyl-2-nitrobenzol | 0,09 | 0,09 |
| 2,4,5,6-Tetraaminopyrimidinsulfat | 1,46 | 1,46 |
| Bis-(5-Amino-2-hydroxyphenyl)methan 2HCl | 0,06 | 0,06 |
| Ammoniumsulfat techn. rein | 0,02 | 0,02 |
| Ascorbinsäure | 0,10 | 0,10 |
| Natriumsulfit wasserfrei | 0,40 | 0,40 |
| HEDP 60% | 0,20 | 0,20 |
| Natriumsilikat 40/42 | 0,50 | 0,50 |
| Ammoniak 25 % | 6,50 | 6,50 |
| Parfum | qs | qs |
| Wasser, vollentsalzt | ad 100 | ad 100 |

c) Entwicklerdispersion EW

| Rohstoff | EW [Gew.-%] |
|---|---|
| Dipicolinsäure | 0,10 |
| Dinatriumpyrophosphat | 0,03 |
| HEDP, 60% | 1,50 |
| Texapon NSO | 2,00 |
| Dow Corning DB 110 A | 0,07 |
| Aculyn 33A | 15,00 |
| Ammoniak, 25 % | 0,65 |
| Wasser, vollentsalzt | ad 100 |

Eingesetzte Rohstoffe:

| | |
|---|---|
| Lanette® D | INCI-Bezeichnung: Cetearyl alcohol (Cognis) |
| Lorol® tech. | INCI-Bezeichnung: Coconut alcohol (Cognis) |
| Eumulgin® B2 | INCI-Bezeichnung: Ceteareth-20 (Cognis) |
| Eumulgin® B1 | INCI-Bezeichnung: Ceteareth-12 (Cognis) |
| Lamesoft® PO 65 | INCI-Bezeichnung: Coco-Glucoside, Glyceryl Oleate, Aqua (Cognis) |
| Akypo® Soft 45HP | INCI-Bezeichnung: Sodium Laureth-6 Carboxylate, Aqua (KAO) |
| Texapon® K14 S Special | INCI-Bezeichnung: Sodium Myreth Sulfate, Aqua (Cognis) |
| Produkt W 37194 | INCI-Bezeichnung: Acrylamidopropyltrimonium Chloride/Acrylates Copolymer, Aqua (Stockhausen) |
| Produkt T 4835 | erfindungsgemäßes amphoteres Tensid gemäß Formel (Ia) mit n+m = 8-12 und R = $C_{14}/C_{16}/C_{18}$-Alkyl; (ca. 20-25 Gew.-% in Wasser) |
| Natriumsilikat 40/42 | Natronwasserglas |
| Texapon® NSO UP | INCI-Bezeichnung: Sodium Laureth Sulfate (Cognis) |
| Aculyn® 33 | INCI-Bezeichnung: Acrylates Copolymer (Rohm & Haas) |
| Dow Corning® DB 110 | INCI-Bezeichnung: Dimethicon (Dow Corning) |

*Herstellung der Färbecremes:*

Lanette D, Lorol, Eumulgin B1, Eumulgin B2 und Lamesoft PO 65 wurden zusammen bei 80 °C aufgeschmolzen und mit einem Teil der Wassermenge dispergiert. Anschließend wurden die restlichen Rezepturbestandteile unter Rühren der Reihe nach eingearbeitet. Dann wurde mit Wasser auf 100 Gew.-% aufgefüllt und die Formulierung kalt gerührt. Die Rezeptur E1 ist ein erfindungsgemäßes Beispiel. Bei der Rezeptur V1 handelt es sich um eine nicht erfindungsgemäße Vergleichsrezeptur erfindungsgemäßes amphoteres Tensid. Die anwendungsbereiten Färbemittel werden durch Vermischen von gleichen Gewichtsanteilen aus der jeweiligen Färbecreme (E1 oder V1) sowie der Entwicklerdispersion EW hergestellt.

## 2. Färbeergebnisse

### *2.1 Egalisiervermögen auf geschädigtem Naturhaar*

2.1.1 Egalisiersträhnen

Die Egalisierung soll eine typische Situation von vorbehandelten Haaren mit einem nachgewachsenen Haaransatz simulieren. Als Haarmaterial wurde Kerling, 80% meliert verwendet. Hierzu wurden diese Strähnen in zwei Bereiche (oberer Teil und unterer Teil) aufgeteilt. Der obere Teil der Strähne blieb unbehandelt, während der untere Teil der Strähnen mit dem Handelsprodukt Poly Blonde L1+ für 30min bei Raumtemperatur blondiert wurde. Anschließend wurden die Strähnen gründlich gespült und getrocknet. Die Strähnen wurden nach dieser Vorschädigung 14 Tage bei Raumtemperatur gelagert, bevor die eigentlichen Versuche durchgeführt wurden.

2.1.2 Färbevorgang

Für die Vorbehandlung wurde auf die vorbereiteten Strähnen von ca. 0,7 g Gewicht die gleiche Menge des Vorbehandlungsmittels (VM1) appliziert. Das Mittel wurde für 5 min auf dem Haar einwirken gelassen, bevor auf die vorbehandelten Strähnen die 4-fache Menge des eigentlichen, anwendungsbereiten Färbemittels (E1+EW sowie V1+EW) aufgetragen wurde. Nachdem die Strähnen für 30 Minuten bei 32 °C gefärbt wurden, wurden sie mit einem handelsüblichen Shampoo gewaschen und mit einem Föhn getrocknet (Versuche # 2 und 4, Tabelle 1).

Für die eigentliche Färbung ohne Vorbehandlung wurde auf die Strähnen von ca. 0,7 g Gewicht jeweils die 4-fache Menge der fertigen Anwendungsmischungen (E1+EW sowie V1+EW) appliziert. Nachdem die Strähnen für 30 Minuten bei 32 °C gefärbt wurden, wurden sie mit einem handelsüblichen Shampoo gewaschen und mit einem Föhn getrocknet (Versuche # 1 und 3, Tabelle 1).

2.1.3 Ergebnisse

Die farbmetrischen Messungen erfolgten auf dem oberen sowie auf dem unteren Teil der Egalisiersträhne an jeweils 4 Messpunkten pro Strähnenhälfte. Als Messgerät diente der Spectralflash SF 450 der Firma Datacolor. Folgende Messparameter wurden dabei verwendet:

mit Glanz; Messblende SAV; D65 (Tageslicht); 10° Beobachter.

Die Ergebnisse der Messungen wurden mithilfe des CIELAB Farbenraums quantifiziert.

Der L-Wert steht dabei für die Helligkeit der Färbung (schwarz-weiß-Achse); je größer der Wert für L ist, desto heller ist die Färbung. Der a-Wert steht für die rot-grün-Achse des Systems; je größer der Wert ist, umso mehr ist die Färbung ins Rote verschoben. Der b-Wert steht für die gelb-blau-Achse des Systems; je größer der Wert ist, umso mehr ist die Färbung ins Gelbe verschoben. Farbunterschiede werden mittels des ΔE-Wertes charakterisiert, der entsprechend Gleichung (I) berechnet wird:

$$\Delta E = \sqrt{(\Delta L)^2 + (\Delta a)^2 + (\Delta b)^2} \qquad (I)$$

Es wurden die folgenden Egalisierungen ermittelt (Tabelle 1):

| Versuch # | Vorbehandlung | Färbemittel | Strähnenteil | L | a | b | ΔE |
|---|---|---|---|---|---|---|---|
| 1 | - | V1+EW | oben | 23,8 | 19,6 | 10,1 | 14,1 |
| | | | unten | 16,9 | 9,3 | 3,4 | |
| 2 | VM1 | V1+EW | oben | 23,8 | 19,9 | 9,4 | 12,3 |
| | | | unten | 17,8 | 10,6 | 4,0 | |
| 3 | - | E1+EW | oben | 23,5 | 21,0 | 10,3 | 9,7 |
| | | | unten | 19,1 | 13,8 | 5,7 | |
| 4 | VM1 | E1+EW | oben | 24,7 | 23,6 | 11,6 | 7,8 |
| | | | unten | 20,9 | 17,8 | 7,9 | |

Ein kleinerer ΔE-Wert bedeutet dabei einen geringeren Farbunterschied zwischen dem unbehandelten, oberen Teil der Strähne und dem vorblondierten, unteren Teil der Strähne. Er belegt eine gleichmäßigere und einheitlichere Färbung der Strähne und somit ein verbessertes Egalisiervermögen des Mittels.

Färbungen, bei denen zuvor eine Vorbehandlung mit einem Mittel, enthaltend das amphotere Tensid gemäß Formel (I) durchgeführt worden war (Versuche 2 und 4), weisen eine verbesserte Egalisierung gegenüber den korrespondierenden, unbehandelten Strähnen (Versuche 1 und 3) auf. Anhand der ΔE-Werte kann insbesondere abgelesen werden, dass erfindungsgemäße Mittel, die das erfindungsgemäße amphotere Tensid in der Färbecreme enthalten (Versuche 3 und 4), eine deutlich verbesserte Egalisierung zwischen geschädigten und ungeschädigten Haarsträhnen gegenüber den Färbemitteln ohne amphoteres Tensid gemäß Formel (I) (Versuche 1 und 2) aufweisen.

### *2.2 Farberhalt*

Für die eigentliche Färbung wurde auf 2 verschiedene Typen Strähnen (Haarmaterial: Kerling, 80% meliert: Versuche # 5 und 6; Kerling, 80% grau, blondiert, s. o., Versuche # 7 und 8) von jeweils ca. 0,7 g Gewicht die 4-fache Menge der fertigen Anwendungsmischungen (E1+EW und V1+EW) appliziert. Nachdem die Strähnen für 30 Minuten bei 32 °C gefärbt wurden, wurden sie mit einem handelsüblichen Shampoo gewaschen und mit einem Föhn getrocknet. Das gefärbte Haar wurde einem Cyclus aus 1 min Waschen mit einem handelsüblichen Shampoo und anschließendem Trocknen mit einem Föhn unterworfen. Dieser Cyclus wurde 6-mal wiederholt.

Für die Berechnung des Farberhalts wurden die ungefärbten Strähnen sowie die gefärbten Strähnen vor und nach den Waschcyclen mit Hilfe des Farbmessgerät der Firma Datacolor, Typ Spectraflash 450 vermessen.

Der Farberhalt FA wird üblicherweise als prozentualer Anteil der ursprünglichen Coloration im Vergleich zur gewaschenen Coloration angegeben und berechnet sich nach folgender Formel:

$$FA = 100 \cdot \left( \frac{\Delta E_{u;g}}{\Delta E_{u;c}} \right) = 100 \cdot \left( \frac{\sqrt{(L_u - L_g)^2 + (a_u - a_g)^2 + (b_u - b_g)^2}}{\sqrt{(L_u - L_c)^2 + (a_u - a_c)^2 + (b_u - b_c)^2}} \right)$$

Der Index u bezeichnet dabei uncoloriertes Haar, der Index c das colorierte, ungewaschene Haar, während der Index g, das colorierte, gewaschene Haar bezeichnet.

Es wurden die folgenden Farberhaltergebnisse ermittelt (Tabelle 2):

| Vers. # | Haartyp | Färbemittel | Strähne | L | a | b | FA [%] |
|---|---|---|---|---|---|---|---|
| | 80% meliert | - | uncoloriert | 66,0 | 5,0 | 20,8 | |
| | 80% meliert, blondiert | - | uncoloriert | 71,5 | 4,5 | 24,5 | |
| 5 | 80% meliert | V1+EW | coloriert | 23,8 | 19,6 | 10,1 | 93,4 |
| | | | coloriert + 6x gewaschen | 27,8 | 23,3 | 13,4 | |
| 6 | 80% meliert | E1+EW | coloriert | 23,5 | 21,0 | 10,3 | 96,8 |
| | | | coloriert + 6x gewaschen | 25,6 | 23,1 | 11,6 | |
| 7 | 80% meliert, blondiert | V1+EW | coloriert | 16,9 | 9,3 | 3,4 | 90,0 |
| | | | coloriert + 6x gewaschen | 22,7 | 19,1 | 10,6 | |
| 8 | 80% meliert, blondiert | E1+EW | coloriert | 19,1 | 13,8 | 5,7 | 94,4 |
| | | | coloriert + 6x gewaschen | 22,3 | 18,8 | 9,8 | |

Die Ergebnisse zeigen auf beiden Haartypen deutlich den verbesserten Farberhalt nach 6-maligen Waschen bei den erfindungsgemäßen Mitteln (# 6 und 8) gegenüber den korrespondieren Mittel ohne amphoteres Tensid (# 5 und 7).

## Patentansprüche

1. Mittel zur Färbung keratinischer Fasern, enthaltend in einem kosmetischen Träger mindestens eine farbgebende Komponente, **dadurch gekennzeichnet, dass** es zusätzlich mindestens eine Verbindung gemäß Formel (I) und/ oder eines ihrer physiologisch verträglichen Salze enthält,

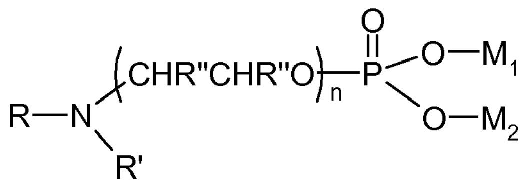

(I),

worin

R für eine $C_8$-$C_{30}$-Alkylgruppe, welche bis zu 4 Doppelbindungen enthalten kann, steht,
R' für ein Wasserstoffatom, eine $C_1$-$C_{30}$-Alkylkette, eine $C_2$-$C_{30}$-Hydroxyalkylkette oder eine Gruppe $(CH_2CH_2O)_m$-Y steht, wobei Y für ein Wasserstoffatom, einen Sulfatester $S(=O)_2OM_3$ oder einen Phosphatester $P(=O)(OM_3)(OM_4)$ steht und m für eine ganze Zahl von 0 bis 50 steht,
R" unabhängig voneinander für ein Wasserstoffatom oder eine $C_1$-$C_4$-Alkylkette steht,
n für eine ganze Zahl von 1 bis 50 steht,
und $M_1$, $M_2$ sowie gegebenenfalls $M_3$ und $M_4$ jeweils unabhängig voneinander für ein Wasserstoffatom, ein Metallatom oder eine gegebenenfalls alkylierte Ammonium-Einheit steht.

2. Mittel nach Anspruch 1, **dadurch gekennzeichnet, dass** in Formel (I) R' für eine Gruppe $(CH_2CH_2O)_m$-Y steht, wobei Y für einen Sulfatester $S(=O)_2OM_3$ oder einen Phosphatester $P(=O)(OM_3)(OM_4)$ und m für eine ganze Zahl von 1 bis 50 steht.

3. Mittel nach einem der Ansprüche 1 oder 2, **dadurch gekennzeichnet, dass** in Formel (I) R für eine $C_{10}$-$C_{22}$-Alkylgruppe, welche bis zu 4 Doppelbindungen enthalten kann, steht.

4. Mittel nach einem der Ansprüche 1 bis 3, **dadurch gekennzeichnet, dass** in Formel (I) R" für ein Wasserstoffatom oder eine Methylgruppe steht und n für eine ganze Zahl von 1 bis 25 steht.

5. Mittel nach einem der Ansprüche 1 bis 4, **dadurch gekennzeichnet, dass** in Formel (I) der Rest R' für eine ethoxylierte Phosphatester Gruppe $(CH_2CH_2O)_m$-$P(=O)(OM_3)(OM_4)$ steht und m für eine ganze Zahl von 1 bis 25 steht.

6. Mittel nach Anspruch 5, **dadurch gekennzeichnet, dass** in Formel (I) R" für ein Wasserstoffatom und n und m jeweils unabhängig voneinander für eine ganze Zahl von 1 bis 25 stehen.

7. Mittel nach einem der Ansprüche 5 oder 6, **dadurch gekennzeichnet, dass** in Formel (I) R" für ein Wasserstoffatom, R für einen Talgalkylrest und die Summe aus n und m eine ganze Zahl zwischen 8 bis 12 stehen.

8. Mittel nach einem der Ansprüche 1 bis 7, **dadurch gekennzeichnet, dass** das Mittel als farbgebende Komponente mindestens ein Oxidationsfarbstoffvorprodukt enthält.

9. Mittel nach einem der Ansprüche 1 bis 8, **dadurch gekennzeichnet, dass** das Mittel als farbgebende Komponente mindestens einen direktziehenden Farbstoff enthält.

10. Mittel nach einem der Ansprüche 1 bis 9, **dadurch gekennzeichnet, dass** das Mittel zusätzlich mindestens ein anionisches Tensid enthält.

11. Mittel nach Anspruch 10, **dadurch gekennzeichnet, dass** das Mittel als zusätzliches anionisches Tensid mindestens eine Ethercarbonsäure der Formel $RO(CH_2CH_2O)_xCH_2COOH$ und/oder eines ihrer physiologisch verträglichen Salze enthält, worin R für eine $C_{10}$-$C_{20}$-Alkylkette steht und x = 0 oder eine Zahl 1 bis 16 ist.

12. Kosmetische Verwendung einer Verbindung gemäß Formel (I) und/oder eines ihrer physiologisch verträglichen Salze zur Verbesserung der Waschbeständigkeit von Färbungen auf keratinischen Fasern.

13. Kosmetische Verwendung einer Verbindung gemäß Formel (I) und/oder eines ihrer physiologisch verträglichen

Salze zur Verbesserung der Egalisierung von Färbungen auf keratinischen Fasern.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 998908 A2


### In der Beschreibung aufgeführte Nicht-Patentliteratur


- **Kh. Schrader.** Grundlagen und Rezepturen der Kosmetika. Hüthig Buch Verlag, 1989